# EUROPEAN PATENT APPLICATION

(11) **EP 4 759 800 A1**
(43) Date of publication of application: **17.06.2026**
(21) Application number: 24851588.4
(22) Date of filing: 23.07.2024
(51) Int. Cl.: C07D 209/58, C08G 59/50

(54) **AMINO GROUP-CONTAINING COMPOUND, CURABLE RESIN COMPOSITION, CURED PRODUCT, AND LAMINATE**

(30) Priority: 10.08.2023 JP 2023131568
(71) Applicant: DIC Corporation, Tokyo 174-8520 (JP); NATIONAL UNIVERSITY CORPORATION YOKOHAMA NATIONAL UNIVERSITY, Yokohama-shi Kanagawa 240-8501 (JP)
(72) Inventor: OYAMA Toshiyuki, Yokohama-shi, Kanagawa 240-8501 (JP); KIMURA Risa, Yokohama-shi, Kanagawa 240-8501 (JP); SUZUKI Etsuko, Sakura-shi, Chiba 285-8668 (JP); ARITA Kazuo, Sakura-shi, Chiba 285-8668 (JP)
(74) Representative: TBK
(86) International application number: PCT/JP2024/026257
(87) International publication number: WO 2025/033159

(57) **Abstract**

Provided are a compound that is a curable resin but can easily achieve repairability and remoldability in a cured article, as well as a curable resin composition including the compound and a cured article thereof. An amino group-containing compound of the present invention is an amino group-containing compound represented by the following general formula (1) and having a molecular weight of less than 1000. 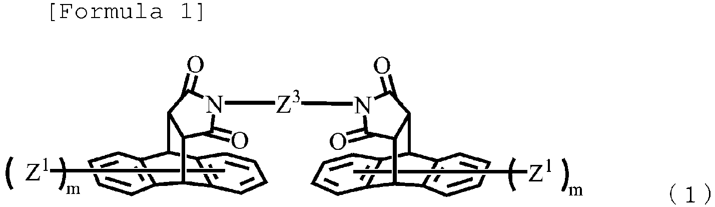 In general formula (1), m is an integer of 1 to 10. Z¹'s are each independently a hydrogen atom, an amino group, a halogen atom, an alkoxy group, an aralkyloxy group, an aryloxy group, a nitro group, an amide group, an alkyloxycarbonyl group, an aryloxycarbonyl group, a cyano group, an alkyl group, a cycloalkyl group, an aralkyl group, or an aryl group, and at least one of Z¹'s is an amino group or a group having an amino group as a substituent. Z³ is any of the structures represented by formulae (2) in the description.

## Description

### Technical Field

The present invention relates to an amino group-containing compound having a specific structure, a curable resin composition containing the compound, a cured article, and a laminated product containing a layer of the cured article.

The present application claims priority based on Japanese Patent Application No. 2023-131568 filed in Japan on August 10, 2023, the contents of which are hereby incorporated herein by reference.

### Background Art

Cured articles obtained from epoxy resins are excellent in heat resistance, mechanical strength, electrical properties, and adhesiveness, and are essential materials in various fields such as electrical and electronic applications, paints, and adhesives.

On the other hand, cured articles using thermosetting resins such as epoxy resins have low long-term reliability. For example, cracks may occur when cured articles of epoxy resins undergo oxidation degradation.

In addition, cured articles obtained once thermosetting resins such as epoxy resins are cured cannot be dissolved in solvents (insoluble) and do not melt at high temperatures (infusible). Because of this, the cured articles are less recyclable and less reusable and become waste after use, and reduction in such waste and environmental load is now at issue.

Cured articles using epoxy resins or the like require problem solutions such as long service life and waste reduction. These solutions can be effectively achieved by imparting ease of disassembly, repairability, and remoldability to the cured articles.

Against this background, a method to enable disassembly has been disclosed, in which a thermally decomposable compound is blended in advance with a reactive adhesive component, so that adhesive strength is reduced by a certain amount of heating after use (see, for example, PTL 1).

Furthermore, a technology related to a sealing material including an epoxy resin or the like and capable of self-repairing cracks and peeling is disclosed, in which the sealing material includes a first thermosetting resin and microcapsule particles containing a second thermosetting resin precursor (see, for example, PTL 2).

In addition to the above, there has been much research on the use of reversible bonding such as dynamic covalent bonding and supramolecular bonding in cured articles to impart repairability and remoldability.

### Citation List

### Patent Literature

PTL 1: Japanese Unexamined Patent Application Publication No. 2013-256557
PTL 2: Japanese Unexamined Patent Application Publication No. 2017-041496

### Summary of Invention

### Technical Problem

In the technology provided in PTL 1, the adhesive is disposed of after disassembly, and although substrates serving as adherends are recyclable, the overall recyclability is insufficient. The technology described in PTL 2 has a certain degree of self-repairability, but it is not a solution from the viewpoint of reuse, and the problem of waste remains when the material becomes unnecessary. Raw materials involved with the reversible bonding must ensure molecular mobility. This limits the raw materials to the use of gel-like materials that lack mechanical strength. In any case, there is a need for improvement at present. An object of the present invention is to provide a compound that is a curable resin but can easily achieve repairability and remoldability in a cured article, as well as a curable resin composition including the compound and a cured article thereof.

### Solution to Problem

As a result of diligent study, the inventors of the present invention have found that the above problem can be solved by using an amino group-containing compound having a specific structure and using the compound as a curable resin composition. This finding has led to completion of the invention.

More specifically, the present invention provides the following aspects.
[1] An amino group-containing compound represented by the following general formula and having a molecular weight of less than 1000: 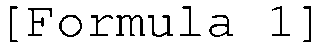 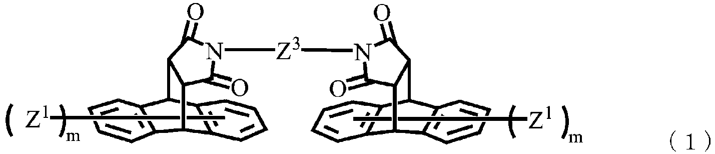
   wherein m is an integer of 1 to 10;
   Z¹'s are each independently a hydrogen atom, an amino group, a halogen atom, an alkoxy group, an aralkyloxy group, an aryloxy group, a nitro group, an amide group, an alkyloxycarbonyl group, an aryloxycarbonyl group, a cyano group, an alkyl group, a cycloalkyl group, an aralkyl group, or an aryl group, and at least one of Z¹'s is an amino group or a group having an amino group as a substituent; and
   Z³ is any of the structures represented by the following formulae (2), 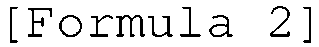 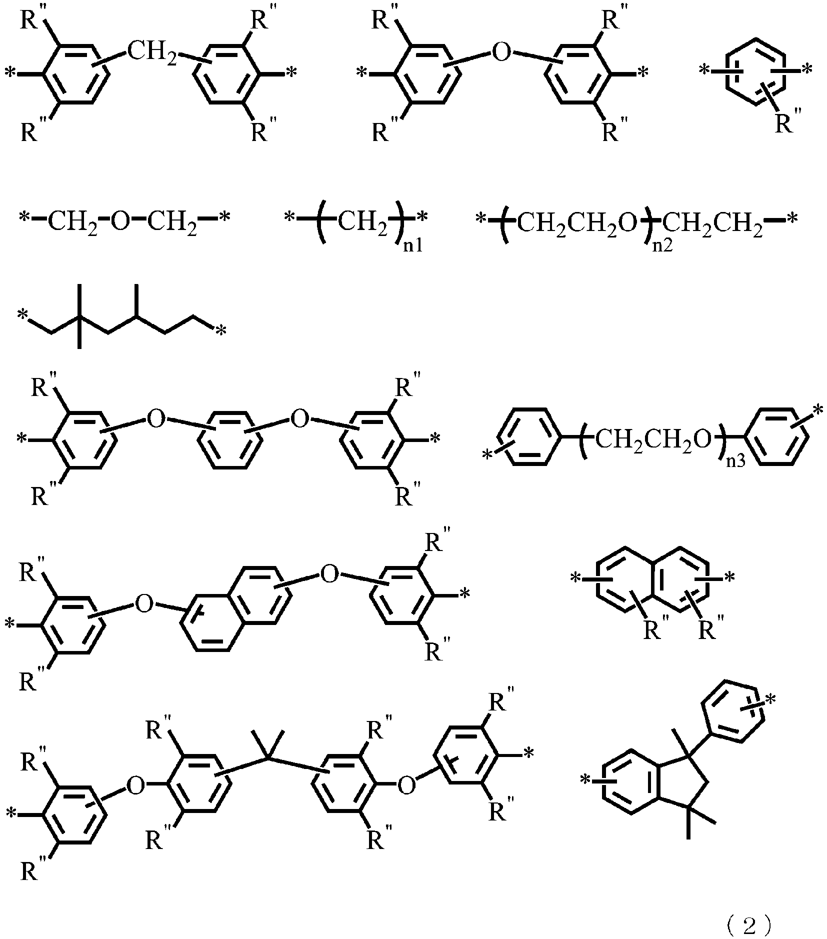
   wherein each R'' is independently a hydrogen atom, a methyl group, or an ethyl group, n1 is an integer of 1 to 30, n2 is an average number of repetitions and is 0.5 to 8, n3 is an average number of repetitions and is 0.5 to 6, and * represents a bonding point.
[2] A curable resin composition including, as essential components, the amino group-containing compound according to [1] and a compound (I) having reactivity with the amino group-containing compound.
[3] The curable resin composition according to [2], wherein a concentration of reversible bonds in the amino group-containing compound to a total mass of curable components in the curable resin composition is 0.10 mmol/g or more.
[4] The curable resin composition according to [2] or [3], wherein the compound (I) having reactivity with the amino group-containing compound is an epoxy resin.
[5] The curable resin composition according to [4], further including a curing agent for epoxy resins other than the amino group-containing compound.
[6] The curable resin composition according to [4] or [5], wherein the epoxy resin is represented by the following formula (3) and has an epoxy equivalent weight of 500 to 10000 g/eq: 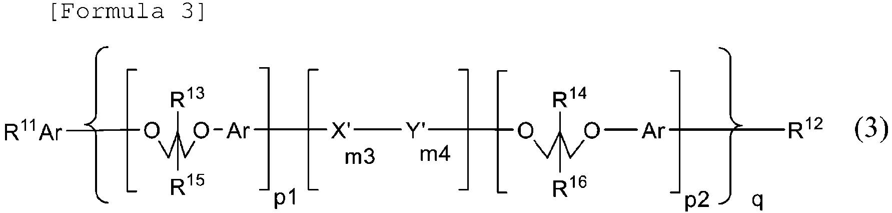
   wherein each Ar is independently a structure having an aromatic ring that is unsubstituted or has a substituent;
   X' is a structural unit represented by the following general formula (3-1), and Y' is a structural unit represented by the following general formula (3-2), 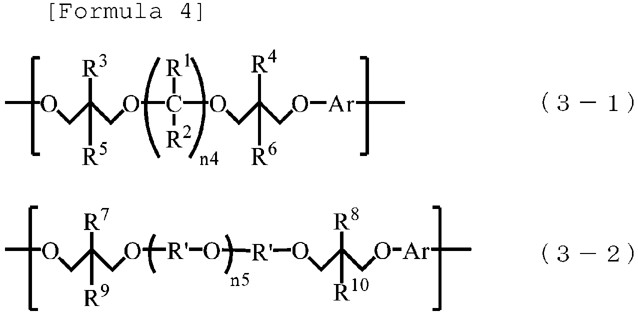
   wherein Ar is as defined above,
   R¹ and R² are each independently a hydrogen atom, a methyl group, or an ethyl group,
   R' is a divalent hydrocarbon group having 2 to 12 carbon atoms,
   R³, R⁴, R⁷, and R⁸ are each independently a hydroxyl group, a glycidyl ether group, or a 2-methylglycidyl ether group,
   R⁵, R⁶, R⁹, and R¹⁰ are each independently a hydrogen atom or a methyl group,
   n4 is an integer of 2 to 16, and
   n5 is an average number of repeating units and is 2 to 30;
   R¹¹ and R¹² are each independently a glycidyl ether group or a 2-methylglycidyl ether group;
   R¹³ and R¹⁴ are each independently a hydroxyl group, a glycidyl ether group, or a 2-methylglycidyl ether group;
   R¹⁵ and R¹⁶ are each a hydrogen atom or a methyl group;
   m3, m4, p1, p2, and q are each an average number of repetitions,
   m3 and m4 are each independently 0 to 25 and m3 + m4 ≥ 1,
   p1 and p2 are each independently 0 to 5, and
   q is 0.5 to 5,
   provided that bonding between X' represented by the general formula (3-1) and Y' represented by the general formula (3-2) is optionally random or block, and total numbers of the structural units X' and Y' in one molecule are m3 and m4, respectively.
[7] The curable resin composition according to [6], wherein the epoxy resin is represented by the following formula (4): 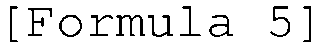 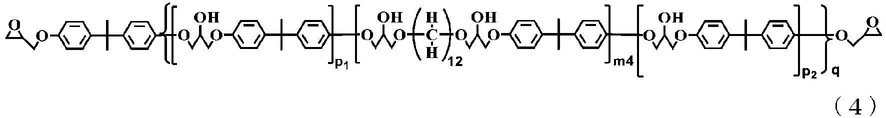 wherein p1, p2, q, and m4 are each an average number of repetitions, and independently, p1 is 0 to 5, p2 is 0 to 5, q is 0.5 to 5, and m4 is 0 to 25.
[8] A curable resin composition, wherein the curable resin composition according to any one of [2] to [7] is a self-repairable composition or a composition for remolding materials.
[9] A cured article made by curing the curable resin composition according to any one of [2] to [7].
[10] A laminated product including: a substrate; and a layer including the cured article according to [9].
[11] A heat-resistant member containing the cured article according to [9].
[12] A method for producing an amino group-containing compound, the method including synthesizing in situ an amino group-containing compound represented by the formula (1) in a process of curing with a compound (I) having reactivity with the amino group-containing compound, using a dienophile intermediate represented by the following general formula (1)': 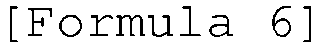 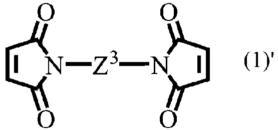
[13] A cured article obtained by a curing reaction using the formula (1)', an anthracene having an amino group, and a compound (I) having reactivity with the amino group-containing compound as essential components.

### Advantageous Effects of Invention

According to the present invention, it is possible to impart repairability and remoldability to the cured article of the curable resin composition, contributing to a longer service life of the cured article itself and reduced waste.

### Brief Description of Drawings

FIG. 1 is a chart illustrating the ¹H-NMR spectrum of an amino group-containing compound obtained in Example 1.
FIG. 2 is a chart illustrating thermogravimetric analysis (TGA) results of epoxy resin cured articles "DGEBA/ABA" and "DGEBA/AA/BMI" obtained in Examples 4 and 5, respectively.
FIG. 3 is a chart illustrating dynamic mechanical analysis (DMA) results of the epoxy resin cured articles "DGEBA/ABA" and "DGEBA/AA/BMI" obtained in Examples 4 and 5, respectively.

### Description of Embodiments

Embodiments for carrying out the present invention will now be described in detail. It should be understood that the present invention is not limited to the following embodiments, and that design changes, improvements, and the like may be made as appropriate based on the ordinary knowledge of those skilled in the art without departing from the spirit of the present invention.

An amino group-containing compound in one aspect of the present invention is a compound represented by the following general formula and having a molecular weight of less than 1000: 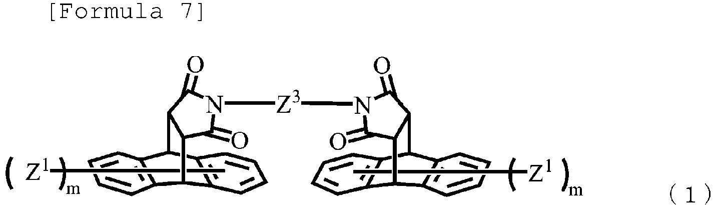
wherein m is an integer of 1 to 10;
Z¹'s are each independently a hydrogen atom, an amino group, a halogen atom, an alkoxy group, an aralkyloxy group, an aryloxy group, a nitro group, an amide group, an alkyloxycarbonyl group, an aryloxycarbonyl group, a cyano group, an alkyl group, a cycloalkyl group, an aralkyl group, or an aryl group, and at least one of Z¹'s is an amino group or a group having an amino group as a substituent; and
Z³ is any of the structures represented by the following formulae (2), 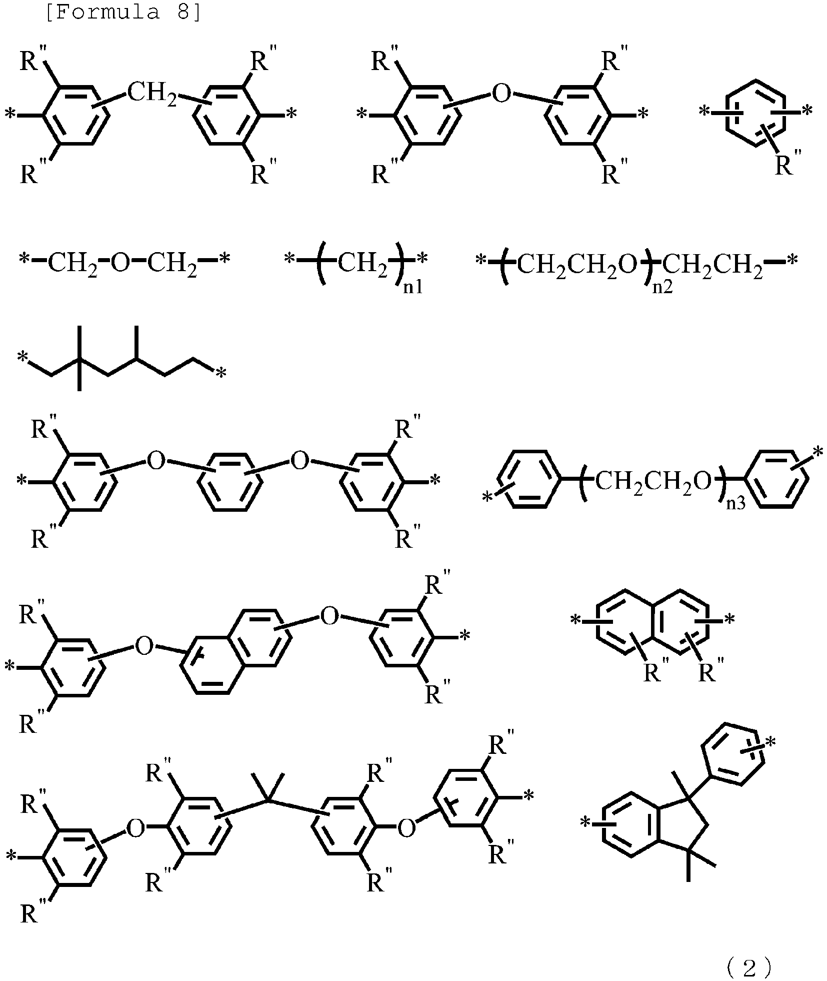
wherein each R'' is independently a hydrogen atom, a methyl group, or an ethyl group, n1 is an integer of 1 to 30, n2 is an average number of repetitions and is 0.5 to 8, n3 is an average number of repetitions and is 0.5 to 6, and * represents a bonding point.

The general formula (1) has a reversible bond formed by an anthracene structure and a maleimide structure at a molecular end. The anthracene structure at an end in general formula (1) has one or more Z¹, in which the amino group contributes to a curing reaction in the curable resin composition described below. m is the number of Z¹'s in the anthracene-derived structure and is an integer of 1 to 10. In terms of the availability of industrial raw materials and the ease of controlling the curing reaction, m is preferably in the range of 1 to 4, and more preferably 1 or 2.

Z¹ in the formula is preferably any of the following structural formulae.

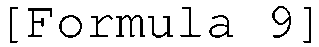

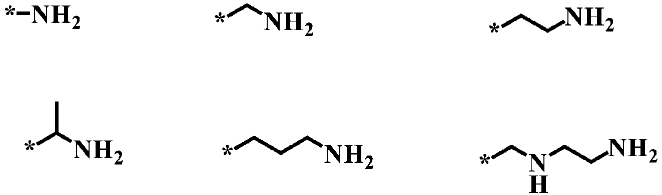

In the structures above, * represents a bonding point.

In the general formula (1), the moiety linking the maleimide-derived structure is Z3, which is any of the structures represented by the general formulae (2).

n1 in the general formula (2) is an integer of 1 to 30, preferably in the range of 1 to 12. n2 is the average number of repetitions, 0.5 to 8, preferably in the range of 2 to 3. n3 is the average number of repetitions, 0.5 to 6, preferably in the range of 2 to 4.

Examples of the amino group-containing compound according to the present invention include, but are not limited to, those represented below.

Among these, a compound with 4,4'-bismaleimidodiphenylmethane skeleton is particularly preferred in terms of repairability and remoldability.

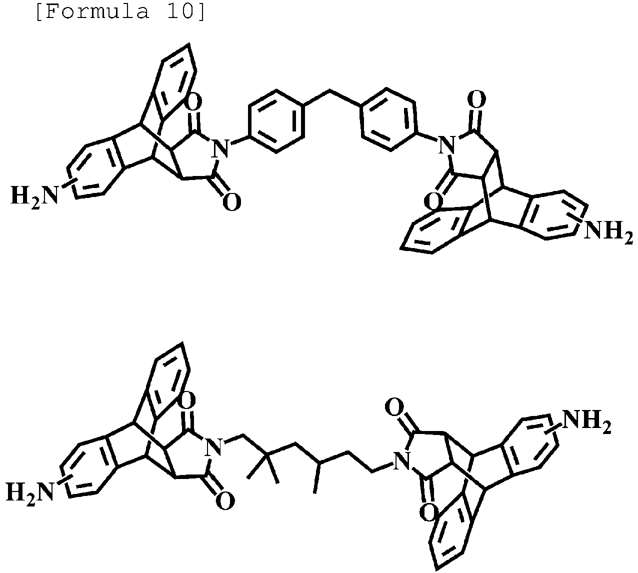

A method for producing an amino group-containing compound according to one embodiment of the present invention is not particularly limited, and depending on the desired structure, the amino group-containing compound can be produced step by step using a known reaction and can be obtained by combining commercially available materials as raw materials as appropriate. Typical synthesis methods are described below.

The general formula (1) has two Diels-Alder reaction units in a molecule. The Diels-Alder reaction unit is an addition reaction part formed as a reversible bond by the Diels-Alder reaction that is made of anthracene and maleimide structures. In general formula (1), the Diels-Alder reaction unit can be obtained by using an anthracene compound having the structure of Z¹.

The so-called Diels-Alder reaction, which is an addition reaction between a conjugated diene such as an anthracene structure and a dienophile such as a maleimide structure to form a six-membered ring, is an equilibrium reaction. At higher temperatures than those at which the addition reaction proceeds, a retro-Diels-Alder reaction proceeds, which is a reverse reaction in which the addition reaction part dissociates back to the original conjugated diene and dienophile. These are widely known.

The anthracene compound having the structure of Z¹ can be any of the compounds listed in the following formulae. Among these, 1-aminoanthracene, 2-aminoanthracene, and 9-aminoanthracene are preferred for good curability, and 1-aminoanthracene and 2-aminoanthracene are particularly preferred for balance of reactivity, cured article properties, and repairability and remoldability. In particular, 1-aminoanthracene is more preferred in terms of the availability of industrial raw materials.

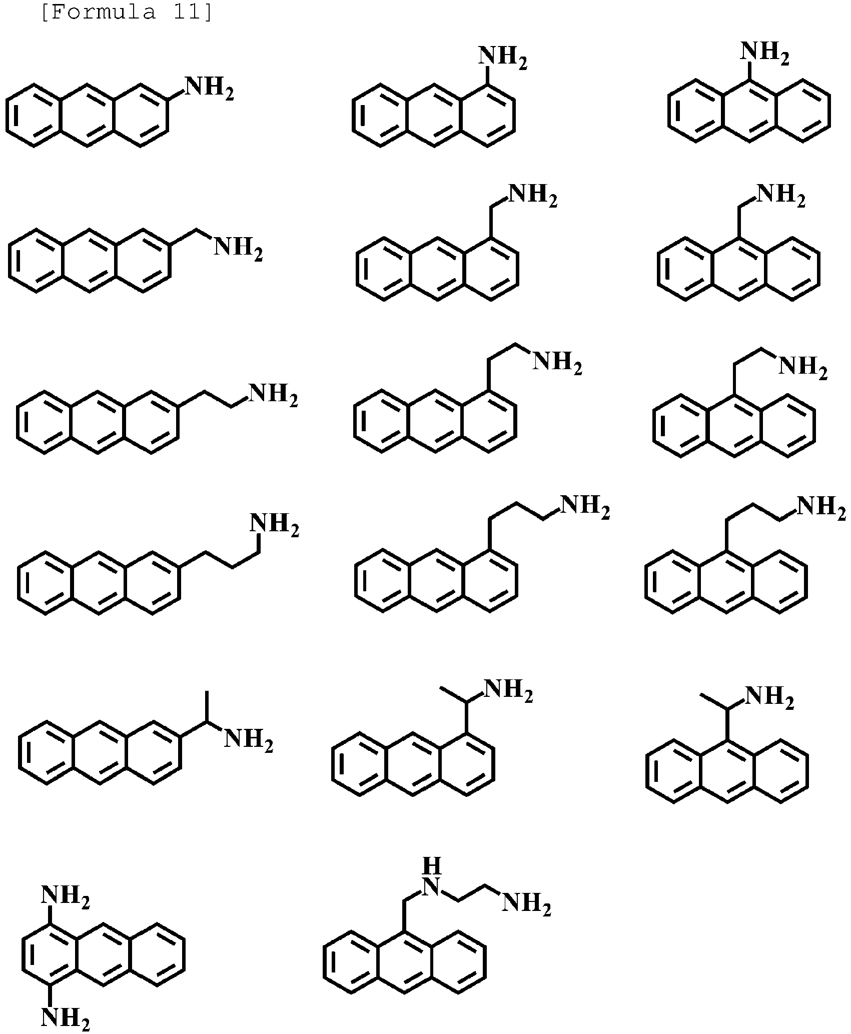

The structures of the maleimide compound and the anthracene compound include those each independently having a hydrogen atom, a halogen atom, an alkoxy group, an aralkyloxy group, an aryloxy group, a nitro group, an amide group, an alkyloxycarbonyl group, an aryloxycarbonyl group, a cyano group, an alkyl group, a cycloalkyl group, an aralkyl group, or an aryl group as a substituent. In the structures of the compounds listed in the above formulae, the alkoxy group, aralkyloxy group, aryloxy group, carboxy group, alkyloxycarbonyl group, aryloxycarbonyl group, alkyl group, cycloalkyl group, aralkyl group, and aryl group also include those having carbon atoms to which various substituents are bonded.

The Diels-Alder reaction can be performed by any known method. For example, a conjugated diene compound and a dienophile compound are mixed in equal molar amounts or in an excess of one of the components in some cases. The mixture is then heated and melted or dissolved in a solvent and stirred at room temperature to 200°C for 1 to 24 hours. Then, the product can be obtained by filtration or solvent removal without purification. Alternatively, the product can be obtained by a commonly used isolation and purification method such as recrystallization, reprecipitation, and chromatography.

A dienophile intermediate before the Diels-Alder reaction can be represented by the following general formula (1)'.

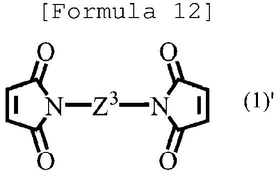

The amino group-containing compound of the present invention can be made into a curable resin composition by combining a compound (I) having reactivity with the amino group-containing compound. The curable resin composition can be suitably used for various electrical and electronic member applications such as adhesives, paints, photoresists, printed circuit boards, and semiconductor sealing materials.

Examples of the compound (I) having reactivity with the amino group-containing compound include melamine compounds substituted with at least one group selected from methylol group, alkoxymethyl group, and acyloxymethyl group, guanamine compounds, glycoluril compounds, urea compounds, resol resins, epoxy resins, isocyanate compounds, azide compounds, compounds containing double bonds such as alkenyl ether group, acid anhydrides, hexamethylenetetramine and its modified products, and oxazoline compounds.

Examples of the melamine compounds include hexamethylolmelamine, hexamethoxymethylmelamine, compounds in which one to six methylol groups of hexamethylolmelamine are methoxymethylated, hexamethoxyethylmelamine, hexaacyloxymethylmelamine, and compounds in which one to six methylol groups of hexamethylolmelamine are acyloxymethylated.

Examples of the guanamine compounds include tetramethylolguanamine, tetramethoxymethylguanamine, tetramethoxymethylbenzoguanamine, compounds in which one to four methylol groups of tetramethylolguanamine are methoxymethylated, tetramethoxyethylguanamine, tetraacyloxyguanamine, and compounds in which one to four methylol groups of tetramethylolguanamine are acyloxymethylated.

Examples of the glycoluril compounds include 1,3,4,6-tetrakis(methoxymethyl)glycoluril, 1,3,4,6-tetrakis(butoxymethyl)glycoluril, and 1,3,4,6-tetrakis(hydroxymethyl)glycoluril.

Examples of the urea compounds include 1,3-bis(hydroxymethyl)urea, 1,1,3,3-tetrakis(butoxymethyl)urea, and 1,1,3,3-tetrakis(methoxymethyl)urea.

Examples of the resol resins include polymers obtained by reacting a phenolic hydroxyl group-containing compound such as phenol, alkylphenol such as cresol and xylenol, phenylphenol, resorcinol, biphenyl, bisphenol such as bisphenol A and bisphenol F, naphthol, or dihydroxynaphthalene with an aldehyde compound under alkaline catalytic conditions.

Examples of the epoxy resins include liquid epoxy resins such as bisphenol A epoxy resin, bisphenol F epoxy resin, bisphenol S epoxy resin, bisphenol AD epoxy resin, polyhydroxybenzene epoxy resin, polyhydroxynaphthalene epoxy resin, biphenyl epoxy resin, and tetramethylbiphenyl epoxy resin, brominated epoxy resins such as brominated phenol novolac epoxy resin, solid bisphenol A epoxy resin, phenol novolac epoxy resin, cresol novolac epoxy resin, triphenylmethane epoxy resin, tetraphenylethane epoxy resin, dicyclopentadiene-phenol addition reaction-type epoxy resin, phenol aralkyl epoxy resin, phenylene ether epoxy resin, naphthylene ether epoxy resin, naphthol novolac epoxy resin, naphthol aralkyl epoxy resin, naphthol-phenol co-condensed novolac epoxy resin, naphthol-cresol co-condensed novolac epoxy resin, aromatic hydrocarbon formaldehyde resin-modified phenol resin epoxy resin, and biphenyl-modified novolac epoxy resin. These may be used alone or in combination of two or more. It is preferable to select and use various types according to the intended use and physical properties of the cured article.

Examples of the isocyanate compounds include tolylene diisocyanate, diphenylmethane diisocyanate, hexamethylene diisocyanate, and cyclohexane diisocyanate.

Examples of the azide compounds include 1,1'-biphenyl-4,4'-bisazide, 4,4'-methylidenebisazide, and 4,4'-oxybisazide.

Examples of the compounds containing double bonds such as alkenyl ether group include ethylene glycol divinyl ether, triethylene glycol divinyl ether, 1,2-propanediol divinyl ether, 1,4-butanediol divinyl ether, tetramethylene glycol divinyl ether, neopentyl glycol divinyl ether, trimethylolpropane trivinyl ether, hexanediol divinyl ether, 1,4-cyclohexanediol divinyl ether, pentaerythritol trivinyl ether, pentaerythritol tetravinyl ether, sorbitol tetravinyl ether, sorbitol pentavinyl ether, and trimethylolpropane trivinyl ether.

Examples of the acid anhydrides include aromatic acid anhydrides such as phthalic anhydride, trimellitic anhydride, pyromellitic anhydride, 3,3',4,4'-benzophenonetetracarboxylic dianhydride, biphenyltetracarboxylic dianhydride, 4,4'-(isopropylidene)diphthalic anhydride, 4,4'-(hexafluoroisopropylidene)diphthalic anhydride; and alicyclic carboxylic acid anhydrides such as tetrahydrophthalic anhydride, methyltetrahydrophthalic anhydride, hexahydrophthalic anhydride, methylhexahydrophthalic anhydride, endomethylenetetrahydrophthalic anhydride dodecenyl succinic anhydride, and trialkyltetrahydrophthalic anhydride.

The concentration of reversible bonds in the curable resin composition of the present invention is preferably 0.10 mmol/g or more relative to the total mass of curable components in the curable resin composition. With such a configuration, both repairability and remoldability of the cured article obtained from the curable resin composition are further improved. The concentration of reversible bonds is more preferably 0.10 to 3.00 mmol/g, and even more preferably 0.15 to 2.00 mmol/g. The concentration of reversible bonds can be selected as appropriate according to a glass transition temperature or the like as defined by the tanδ curve peak top of the target cured article obtained by dynamic mechanical analysis (DMA). For example, when the glass transition temperature is used as a guide, sufficient repairability and remoldability functions are likely to be expressed even at low concentration in a preferred range if the glass transition temperature of the cured article is near room temperature. On the other hand, if the glass transition temperature of the target cured article is above 100°C as a guide, the functions are more likely to be expressed at high concentration in the preferred range. However, in a temperature range above the glass transition temperature measured by DMA, molecular mobility is generally high, and sufficient repairability and remoldability functions are likely to be expressed even with a low concentration of the amino group-containing compound. Based on this, the effect of expressing repairability and remoldability functions can also be adjusted, for example, by appropriately adjusting aging temperature for repair and heating temperature for remolding. Thus, the relation between the glass transition temperature of the cured article and the concentration of reversible bonds is not limited to these.

As the compound (I) having reactivity with the amino group-containing compound, an epoxy resin is particularly preferred in order to produce a curable resin composition with excellent curability, and mechanical strength and heat resistance in the cured article.

As the epoxy resin, an epoxy resin represented by the following formula (3) and having an epoxy equivalent weight of 500 to 10000 g/eq may be used. 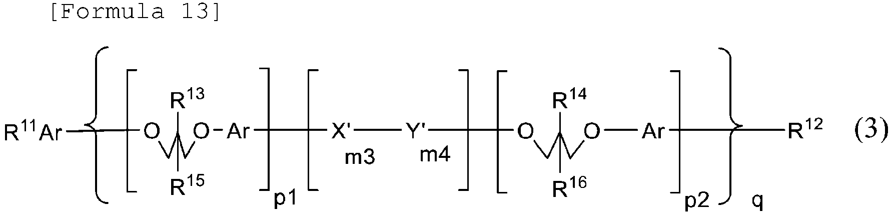
wherein each Ar is independently a structure having an aromatic ring that is unsubstituted or has a substituent;
X' is a structural unit represented by the following general formula (3-1), and Y' is a structural unit represented by the following general formula (3-2), 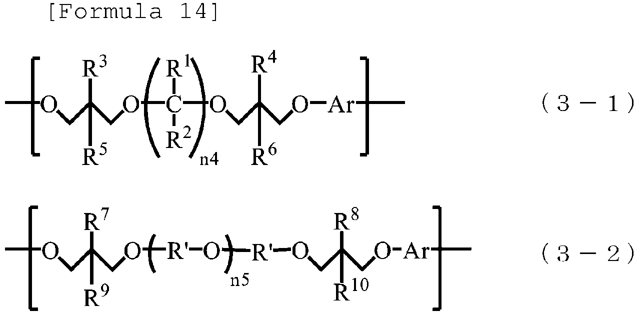
wherein Ar is as defined above,
R¹ and R² are each independently a hydrogen atom, a methyl group, or an ethyl group,
R' is a divalent hydrocarbon group having 2 to 12 carbon atoms,
R³, R⁴, R⁷, and R⁸ are each independently a hydroxyl group, a glycidyl ether group, or a 2-methylglycidyl ether group,
R⁵, R⁶, R⁷, and R¹⁰ are each independently a hydrogen atom or a methyl group,
n4 is an integer of 2 to 16, and
n5 is an average number of repeating units and is 2 to 30;
R¹¹ and R¹² are each independently a glycidyl ether group or a 2-methylglycidyl ether group;
R¹³ and R¹⁴ are each independently a hydroxyl group, a glycidyl ether group, or a 2-methylglycidyl ether group;
R¹⁵ and R¹⁶ are each a hydrogen atom or a methyl group;
m3, m4, p1, p2, and q are each an average number of repetitions,
m3 and m4 are each independently 0 to 25 and m3 + m4 ≥ 1,
p1 and p2 are each independently 0 to 5, and
q is 0.5 to 5,
provided that bonding between X' represented by the general formula (3-1) and Y' represented by the general formula (3-2) is optionally random or block, and total numbers of the structural units X' and Y' in one molecule are m3 and m4, respectively.

As the epoxy resin, an epoxy resin represented by the following formula (4) may be used. The use of such an epoxy resin improves the effect of repairability and remoldability of the epoxy resin cured article and provides a good balance between flexibility and toughness. 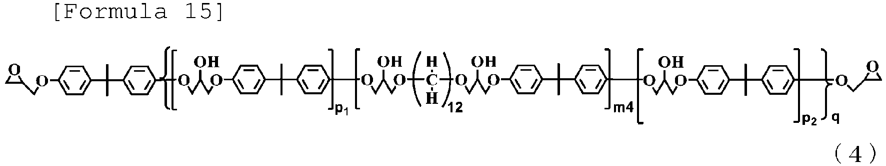 wherein p1, p2, q, and m4 are each an average number of repetitions, and independently, p1 is 0 to 5, p2 is 0 to 5, q is 0.5 to 5, and m4 is 0 to 25.

The epoxy resin represented by the general formula (3) or (4) may be used alone in combination with the amino group-containing compound of the present invention to form a curable resin. It is also preferable to further combine an epoxy resin having an epoxy equivalent weight of 100 to 300 g/eq in order to impart more flexibility to the cured article and easily express repairability and remoldability.

The epoxy resin that can be combined is any epoxy resin that has an epoxy equivalent weight in the range of 100 to 300 g/eq and its structure is not limited. Examples include liquid epoxy resins such as bisphenol A epoxy resin, bisphenol F epoxy resin, bisphenol S epoxy resin, bisphenol AD epoxy resin, polyhydroxybenzene epoxy resin, polyhydroxynaphthalene epoxy resin, biphenyl epoxy resin, and tetramethylbiphenyl epoxy resin, brominated epoxy resins such as brominated phenol novolac epoxy resins, solid bisphenol A epoxy resin, phenol novolac epoxy resin, cresol novolac epoxy resin, triphenylmethane epoxy resin, tetraphenylethane epoxy resin, dicyclopentadiene-phenol addition reaction-type epoxy resin, phenol aralkyl epoxy resin, phenylene ether epoxy resin, naphthylene ether epoxy resin, naphthol novolac epoxy resin, naphthol aralkyl epoxy resin, naphthol-phenol co-condensed novolac epoxy resin, naphthol-cresol co-condensed novolac epoxy resin, aromatic hydrocarbon formaldehyde resin-modified phenol resin epoxy resin, and biphenyl-modified novolac epoxy resin. These may be used alone or in combination of two or more. It is preferable to select and use various types according to the intended use and physical properties of the cured article.

Among these, an epoxy resin having an epoxy equivalent weight of 100 to 300 g/eq is preferred among liquid epoxy resins such as bisphenol A epoxy resin, bisphenol F epoxy resin, bisphenol S epoxy resin, bisphenol AD epoxy resin, polyhydroxybenzene epoxy resin, polyhydroxynaphthalene epoxy resin, biphenyl epoxy resin, and tetramethylbiphenyl epoxy resin. An epoxy resin having an epoxy equivalent weight of 100 to 300 g/eq is particularly preferred among bisphenol A epoxy resin, bisphenol F epoxy resin, bisphenol S epoxy resin, and bisphenol AD epoxy resin.

The ratio of the epoxy resin represented by the general formula (3) or (4) and the epoxy resin having an epoxy equivalent weight of 100 to 300 g/eq is not limited, but from the viewpoint of easy phase separation in the cured article, the mass ratio of the former to the latter is 97:3 to 3:97, preferably 90:10 to 10:90, and particularly preferably 80:20 to 20:80. The phase separation in the cured article results in a sea-island structure, whereby both adhesiveness and stress relaxation ability are achieved in the cured article, in particular, high adhesive strength is expressed in a wide temperature range, and mold shrinkage before and after heat curing of the resin composition is reduced.

Furthermore, when the amino group-containing compound of the present invention is combined with an epoxy resin to form a curable resin composition, a curing agent for epoxy resins other than the amino group-containing compound may be blended.

Examples of the curing agent that can be used herein include various known curing agents for epoxy resins, such as amine compounds, acid anhydrides, amide compounds, phenolic hydroxyl group-containing compounds, carboxylic acid compounds, and thiol compounds.

Examples of the amine compounds include aliphatic amine compounds such as trimethylenediamine, ethylenediamine, N,N,N',N'-tetramethylethylenediamine, pentamethyldiethylenetriamine, triethylenediamine, dipropylenediamine, N,N,N',N'-tetramethylpropylenediamine, tetramethylenediamine, pentanediamine, hexamethylenediamine, trimethylhexamethylenediamine, N,N,N',N'-tetramethylhexamethylenediamine, N,N-dimethylcyclohexylamine, diethylenetriamine, triethylenetetramine, tetraethylenepentamine, dimethylaminopropylamine, diethylaminopropylamine, dibutylaminopropylamine, 1,4-diazabicyclo[2,2,2]octane(triethylenediamine), polyoxyethylenediamine, polyoxypropylenediamine, bis(2-dimethylaminoethyl) ether, dimethylaminoethoxy ethoxyethanol, triethanolamine, dimethylaminohexanol, benzylmethylamine, dimethylbenzylamine, m-xylenediamine, and α-methylbenzylmethylamine;
alicyclic and heterocyclic amine compounds such as piperidine, piperazine, menthanediamine, isophoronediamine, methylmorpholine, ethylmorpholine, N,N',N''-tris(dimethylaminopropyl)hexahydro-s-triazine, a 3,9-bis(3-aminopropyl)-2,4,8,10-tetraoxaspiro[5,5]undecane adduct, N-aminoethylpiperazine, trimethylaminoethylpiperazine, bis(4-aminocyclohexyl)methane, N,N'-dimethylpiperazine, and 1,8-diazabicyclo-[5.4.0]-undecene (DBU);
aromatic amine compounds such as o-phenylenediamine, m-phenylenediamine, p-phenylenediamine, diaminodiphenylmethane, diaminodiphenyl sulfone, pyridine, and picoline; and
modified amine compounds such as an epoxy compound-added polyamine, a polyamine obtained by Michael addition, a polyamine obtained by Mannich addition, a thiourea-added polyamine, a ketone-capped polyamine, dicyandiamide, guanidine, an organic acid hydrazide, diaminomaleonitrile, amineimide, a boron trifluoride-piperidine complex, and a boron trifluoride-monoethylamine complex.

Examples of the acid anhydrides include phthalic anhydride, trimellitic anhydride, pyromellitic anhydride, maleic anhydride, maleic anhydride polypropylene glycol, tetrahydrophthalic anhydride, methyltetrahydrophthalic anhydride, methyl nadic anhydride, hexahydrophthalic anhydride, and methylhexahydrophthalic anhydride.

Examples of the phenolic hydroxyl group-containing compounds include bisphenols such as bis(4-hydroxyphenyl)methane, 2,2-bis(4-hydroxyphenyl)propane, 2,2-bis(3-methyl-4-hydroxyphenyl)propane, 1,1-bis(4-hydroxyphenyl)cyclohexane, and 1,1-bis(4-hydroxyphenyl)-1-phenylethane, and bis(4-hydroxyphenyl)sulfone; and polyhydric phenol compounds such as a phenol novolac resin, a cresol novolac resin, an aromatic hydrocarbon formaldehyde resin-modified phenolic resin, a dicyclopentadiene phenol-added resin, a phenol aralkyl resin (xylok resin), a naphthol aralkyl resin, a trimethylolmethane resin, a tetraphenylolethane resin, a naphthol novolac resin, a naphthol-phenol co-condensed novolac resin, a naphthol-cresol co-condensed novolac resin, a biphenyl-modified phenolic resin (a polyhydric phenol compound having a phenolic nucleus linked through a bismethylene group), a biphenyl-modified naphthol resin (a polyhydric naphthol compound having a phenolic nucleus linked through a bismethylene group), an aminotriazine-modified phenolic resin (a polyhydric phenol compound having a phenolic nucleus linked through melamine, benzoguanamine, or the like), and an alkoxy group-containing aromatic ring-modified novolac resin (a polyhydric phenol compound in which a phenolic nucleus and an alkoxy group-containing aromatic ring are linked through formaldehyde).

Examples of the amide compounds include dicyandiamide and polyamidoamines. Examples of the polyamidoamines include polyamidoamines obtained by a reaction of an aliphatic dicarboxylic acid such as succinic acid, glutaric acid, adipic acid, pimelic acid, suberic acid, or azelaic acid, or a carboxylic acid compound such as a fatty acid or a dimer acid with an aliphatic polyamine or a polyamine having a polyoxyalkylene chain.

Examples of the carboxylic acid compounds include carboxylic acid polymers such as carboxylic acid-terminated polyester, polyacrylic acid, and maleic acid-modified polypropylene glycol.

The thiol compound is preferably a compound containing two or more thiol groups in one molecule. Examples include 3,3'-dithiodipropionic acid, trimethylolpropane tris(thioglycolate), pentaerythritol tetrakis(thioglycolate), ethylene glycol dithioglycolate, 1,4-bis(3-mercaptobutyryloxy)butane, tris[(3-mercaptopropionyloxy)-ethyl]-isocyanurate, trimethylolpropane tris(3-mercaptopropionate), pentaerythritol tetrakis(3-mercaptopropionate), pentaerythritol tetrakis(3-mercaptobutyrate), dipentaerythritol hexakis(3-mercaptopropionate), 1,3,4,6-tetrakis(2-mercaptoethyl)glycoluril, 4-butanedithiol, 1,6-hexanedithiol, and 1,10-decanedithiol.

When these curing agents are used, one type of the curing agents may be used alone, or two or more types thereof may be mixed. For applications such as underfill materials and general coating applications, it is preferable to use the amine compounds, the carboxylic acid compounds, and/or the acid anhydride compounds. For adhesive and flexible wiring board applications, the amine compounds, especially dicyandiamide, are preferred in terms of workability, curability, and long-term stability. For semiconductor sealing material applications, solid-type phenol compounds are preferred in terms of heat resistance of the cured article. For battery applications, aliphatic amines and thiol compounds are preferred in terms of low-temperature curing.

The amounts of the epoxy resin and the curing agent used are not particularly limited, but in terms of good mechanical properties of the resulting cured article, the preferred amounts are such that the amount of an active group that can react with the epoxy group, including the amino group-containing compound of the present invention, is preferably 0.4 to 1.5 equivalents per equivalent of a total of epoxy groups in the resin composition.

When an epoxy resin is used, the curable resin composition may contain a curing accelerator. As the curing accelerator, various compounds can be used. Examples thereof include urea compounds, phosphorus compounds, tertiary amines, imidazoles, imidazolines, organic acid metal salts, Lewis acids, and amine complex salts. For use in adhesive applications, urea compounds, especially 3-(3,4-dichlorophenyl)-1,1-dimethylurea (DCMU), are preferred since they are excellent in workability and low-temperature curability. For use in semiconductor sealing material applications, triphenyl phosphine as a phosphorus compound and 1,8-diazabicyclo-[5.4.0]-undecene as a tertiary amine are preferred since they are excellent in curability, heat resistance, electrical characteristics, moisture-resistant reliability, and the like.

Examples of the phosphorus compounds include alkyl phosphines such as ethylphosphine and butylphosphine, primary phosphines such as phenylphosphine; dialkyl phosphines such as dimethylphosphine and dipropylphosphine; secondary phosphines such as diphenylphosphine and methylethylphosphine; and tertiary phosphines such as trimethylphosphine, triethylphosphine, and triphenylphosphine.

Examples of the imidazoles include imidazole, 1-methylimidazole, 2-methylimidazole, 3-methylimidazole, 4-methylimidazole, 5-methylimidazole, 1-ethylimidazole, 2-ethylimidazole, 3-ethylimidazole, 4-ethylimidazole, 5-ethylimidazole, 1-n-propylimidazole, 2-n-propylimidazole, 1-isopropylimidazole, 2-isopropylimidazole, 1-n-butylimidazole, 2-n-butylimidazole, 1-isobutylimidazole, 2-isobutylimidazole, 2-undecyl-1H-imidazole, 2-heptadecyl-1H-imidazole, 1,2-dimethylimidazole, 1,3-dimethylimidazole, 2,4-dimethylimidazole, 2-ethyl-4-methylimidazole, 1-phenylimidazole, 2-phenyl-1H-imidazole, 4-methyl-2-phenyl-1H-imidazole, 2-phenyl-4-methylimidazole, 1-benzyl-2-methylimidazole, 1-benzyl-2-phenylimidazole, 1-cyanoethyl-2-methylimidazole, 1-cyanoethyl-2-ethyl-4-methylimidazole, 1-cyanoethyl-2-undecylimidazole, 1-cyanoethyl-2-phenylimidazole, a 2-phenylimidazole isocyanuric acid adduct, a 2-methylimidazole isocyanuric acid adduct, 2-phenyl-4,5-dihydroxymethylimidazole, 2-phenyl-4-methyl-5-hydroxymethylimidazole, 1-cyanoethyl-2-phenyl-4,5-di(2-cyanoethoxy)methylimidazole, 1-dodecyl-2-methyl-3-benzylimidazolium chloride, and 1-benzyl-2-phenylimidazole hydrochloride.

Examples of the imidazoline compounds include 2-methylimidazoline and 2-phenylimidazoline.

Examples of the urea compounds include p-chlorophenyl-N,N-dimethyl urea, 3-phenyl-1,1-dimethyl urea, 3-(3,4-dichlorophenyl)-N,N-dimethyl urea, and N-(3-chloro-4-methylphenyl)-N',N'-dimethyl urea.

The curable resin composition of the present invention may be used in combination with other thermosetting resins or thermoplastic resins as long as the effects of the present invention are not impaired.

Examples of other thermosetting resins include cyanate ester resins, resins having benzoxazine structures, active ester resins, vinyl benzyl compounds, acrylic compounds, and copolymers of styrene and maleic anhydride. When the other thermosetting resins described above are used in combination, the amount used is not limited as long as the effects of the present invention are not impaired, but preferably in the range of 1 to 50 parts by mass in 100 parts by mass of the curable resin composition.

Examples of the cyanate ester resins include bisphenol A cyanate ester resin, bisphenol F cyanate ester resin, bisphenol E cyanate ester resin, bisphenol S cyanate ester resin, bisphenol sulfide cyanate ester resin, phenylene ether cyanate ester resin, naphthylene ether cyanate ester resin, biphenyl cyanate ester resin, tetramethylbiphenyl cyanate ester resin, polyhydroxynaphthalene cyanate ester resin, phenol novolac cyanate ester resin, cresol novolac cyanate ester resin, triphenylmethane cyanate ester resin, tetraphenylethane cyanate ester resin, dicyclopentadiene-phenol addition reaction-type cyanate ester resin, phenol aralkyl cyanate ester resin, naphthol novolac cyanate ester resin, naphthol aralkyl cyanate ester resin, naphthol-phenol co-condensed novolac cyanate ester resin, naphthol-cresol co-condensed novolac cyanate ester resin, aromatic hydrocarbon formaldehyde resin-modified phenolic resin cyanate ester resin, biphenyl modified novolac cyanate ester resin, and anthracene cyanate ester resin. These may be each used alone, or two or more types thereof may be used in combination.

Among these cyanate ester resins, bisphenol A cyanate ester resin, bisphenol F cyanate ester resin, bisphenol E cyanate ester resin, polyhydroxynaphthalene cyanate ester resin, naphthylene ether cyanate ester resin, and novolac cyanate ester resin are preferred in order to obtain a cured article with excellent heat resistance. Dicyclopentadiene-phenol addition reaction-type cyanate ester resin is preferred in order to obtain a cured article with excellent dielectric properties.

Examples of the resins having benzoxazine structures include, but are not limited to, a reaction product of bisphenol F with formalin and aniline (F-a type benzoxazine resin), a reaction product of diaminodiphenylmethane with formalin and phenol (P-d type benzoxazine resin), a reaction product of bisphenol A with formalin and aniline, a reaction product of dihydroxydiphenyl ether with formalin and aniline, a reaction product of diaminodiphenyl ether with formalin and phenol, a reaction product of dicyclopentadiene phenol-added resin with formalin and aniline, a reaction product of phenolphthalein with formalin and aniline, and a reaction product of diphenyl sulfide with formalin and aniline. These may be each used alone, or two or more types thereof may be used in combination.

Preferred examples of the active ester resins that can be used generally include, but are not limited to, compounds having two or more ester groups with high reaction activity in one molecule, such as phenol esters, thiophenol esters, N-hydroxyamine esters, and esters of heterocyclic hydroxy compounds. The active ester resin is preferably an active ester resin obtained by a condensation reaction of a carboxylic acid compound and/or a thiocarboxylic acid compound with a hydroxy compound and/or a thiol compound. From the viewpoint of heat resistance improvement, an active ester resin obtained from a carboxylic acid compound or a halide thereof and a hydroxy compound is preferred. An active ester resin obtained from a carboxylic acid compound or a halide thereof and a phenol compound and/or a naphthol compound is more preferred. Examples of the carboxylic acid compound include benzoic acid, acetic acid, succinic acid, maleic acid, itaconic acid, phthalic acid, isophthalic acid, terephthalic acid, pyromellitic acid, and halides thereof. Examples of the phenol compound or the naphthol compound include hydroquinone, resorcin, bisphenol A, bisphenol F, bisphenol S, dihydroxydiphenyl ether, phenolphthalein, methylated bisphenol A, methylated bisphenol F, methylated bisphenol S, phenol, o-cresol, m-cresol, p-cresol, catechol, α-naphthol, β-naphthol, 1,5-dihydroxynaphthalene, 1,6-dihydroxynaphthalene, 2,6-dihydroxynaphthalene, dihydroxybenzophenone, trihydroxybenzophenone, tetrahydroxybenzophenone, phloroglucin, benzenetriol, and dicyclopentadiene phenol-added resin.

As the active ester resin, specifically, an active ester resin containing a dicyclopentadiene-phenol addition structure, an active ester resin containing a naphthalene structure, an active ester resin that is acetylated phenol novolac, an active ester resin that is a benzoylated phenol novolac, and the like are preferred. Among these, the active ester resin containing a dicyclopentadiene-phenol addition structure and the active ester resin containing a naphthalene structure are more preferred since they are excellent in improving peel strength.

Furthermore, various novolac resins, addition polymerization resins of alicyclic diene compounds such as dicyclopentadiene and phenol compounds, modified novolac resins of phenolic hydroxyl group-containing compounds and alkoxy group-containing aromatic compounds, phenol aralkyl resin (xylok resin), naphthol aralkyl resin, trimethylolmethane resin, tetraphenylolethane resin, biphenyl-modified phenol resin, biphenyl-modified naphthol resin, aminotriazine-modified phenol resin, and various vinyl polymers may be used in combination.

More specifically, examples of the various novolac resins include polymers obtained by reacting a phenolic hydroxyl group-containing compound such as phenol, phenylphenol, resorcinol, biphenyl, bisphenol such as bisphenol A and bisphenol F, naphthol, or dihydroxynaphthalene with an aldehyde compound under acid catalytic conditions.

Examples of the various vinyl polymers include homopolymers or copolymers of vinyl compounds such as polyhydroxystyrene, polystyrene, polyvinylnaphthalene, polyvinylanthracene, polyvinylcarbazole, polyindene, polyacenaphthylene, polynorbornene, polycyclodecene, polytetracyclododecene, polynortricyclene, and poly(meth)acrylate.

The thermoplastic resins are resins capable of melt-molding by heating. Specific examples of the thermoplastic resins include polyethylene resin, polypropylene resin, polystyrene resin, rubber-modified polystyrene resin, acrylonitrile-butadiene-styrene (ABS) resin, acrylonitrile-styrene (AS) resin, polymethyl methacrylate resin, acrylic resin, polyvinyl chloride resin, polyvinylidene chloride resin, polyethylene terephthalate resin, ethylene vinyl alcohol resin, cellulose acetate resin, ionomer resin, polyacrylonitrile resin, polyamide resin, polyacetal resin, polybutylene terephthalate resin, polylactic acid resin, polyphenylene ether resin, modified polyphenylene ether resin, polycarbonate resin, polysulfone resin, polyphenylene sulfide resin, polyetherimide resin, polyethersulfone resin, polyarylate resin, thermoplastic polyimide resin, polyamideimide resin, polyetheretherketone resin, polyketone resin, liquid crystal polyester resin, fluororesin, syndiotactic polystyrene resin, and cyclic polyolefin resin. These thermoplastic resins may be used alone or in combination of two or more.

When these other resins are used, the blending ratio between the amino group-containing compound of the present invention and the other resins can be set as desired according to the applications. In order to prevent impairment of repairability and remoldability of the present invention, the proportion of the other resins is preferably 0.5 to 100 parts by mass relative to 100 parts by mass of the amino group-containing compound of the present invention.

The curable resin composition of the present invention may be used in combination with a curing accelerator. Examples of the curing accelerator include tertiary amine compounds such as imidazole and dimethylaminopyridine; phosphorus compounds such as triphenylphosphine; boron trifluoride and boron trifluoride amine complex such as boron trifluoride monoethylamine complex; organic acid compounds such as thiodipropionic acid; benzoxazine compounds such as thiodiphenol benzoxazine and sulfonyl benzoxazine; and sulfonyl compounds. These may be each used alone, or two or more types thereof may be used in combination. The amount of these catalysts added is preferably in the range of 0.001 to 15 parts by mass in 100 parts by mass of the curable resin composition.

When the curable resin composition of the present invention is used for applications that require high flame retardancy, a halogen free flame retardant substantially containing no halogen atom may be blended.

Examples of the halogen free flame retardant include a phosphorus-containing flame retardant, a nitrogen-containing flame retardant, a silicone-based flame retardant, an inorganic flame retardant, and an organic metal salt-based flame retardant. During use of the halogen free flame retardant, there are no limits. The halogen free flame retardant may be used alone, a plurality of halogen free flame retardants of the same type may be used, or halogen free flame retardants of different types may be used in combination.

As the phosphorus-containing flame retardant, an inorganic or organic phosphorus compound may be used. Examples of the inorganic phosphorus compound include red phosphorus, ammonium phosphates such as monoammonium phosphate, diammonium phosphate, triammonium phosphate, and ammonium polyphosphate, and inorganic nitrogen-containing phosphorus compounds such as phosphoric amide.

It is preferable that the red phosphorus is surface-treated to prevent hydrolysis and the like. Examples of a surface treatment method include the following (i) to (iii):
(i) a method for performing a covering treatment with an inorganic compound such as magnesium hydroxide, aluminum hydroxide, zinc hydroxide, titanium hydroxide, bismuth oxide, bismuth hydroxide, bismuth nitrate, or a mixture thereof;
(ii) a method for performing a covering treatment with a mixture of an inorganic compound such as magnesium hydroxide, aluminum hydroxide, zinc hydroxide, or titanium hydroxide, and a thermosetting resin such as a phenol resin; and
(iii) a method for performing a two-stage covering treatment in which a coating of an inorganic compound such as magnesium hydroxide, aluminum hydroxide, zinc hydroxide, or titanium hydroxide is covered with a thermosetting resin such as a phenol resin.

Examples of the organic phosphorus compound include common organic phosphorus compounds such as a phosphoric acid ester compound, a phosphonic acid compound, a phosphinic acid compound, a phosphine oxide compound, a phosphorane compound, and an organic nitrogen-containing phosphorus compound, as well as cyclic organic phosphorus compounds such as 9,10-dihydro-9-oxa-10-phosphaphenanthrene-10-oxide, 10-(2,5-dihydroxyphenyl)-10H-9-oxa-10-phosphaphenanthrene-10-oxide, and 10-(2,7-dihydroxynaphthyl)-10H-9-oxa-10-phosphaphenanthrene-10-oxide, and derivatives thereof obtained by reacting these phosphorus compounds with compounds such as epoxy resins and phenol resins.

The amount of the phosphorus-containing flame retardant blended is appropriately selected depending on the type of phosphorus-containing flame retardant, the other component of the resin composition, and a desired degree of flame retardancy. For example, when red phosphorus is used as a halogen free flame retardant, it is preferably blended in an amount of 0.1 parts by mass to 2.0 parts by mass in 100 parts by mass of the resin composition in which the halogen free flame retardant, filler and other additives are all blended. When the organic phosphorus compound is used, it is preferably blended in an amount of 0.1 parts by mass to 10.0 parts by mass, and more preferably in an amount of 0.5 to 6.0 parts by mass, similarly.

When the phosphorus-containing flame retardant is used, hydrotalcite, magnesium hydroxide, a boron compound, zirconium oxide, a black dye, calcium carbonate, zeolite, zinc molybdate, activated carbon, or the like may be used in combination with the phosphorus-containing flame retardant.

Examples of the nitrogen-containing flame retardant include a triazine compound, a cyanuric acid compound, an isocyanuric acid compound, and phenothiazine. The triazine compound, the cyanuric acid compound, and the isocyanuric acid compound are preferred.

Examples of the triazine compound include melamine, acetoguanamine, benzoguanamine, melon, melam, succinoguanamine, ethylenedimelamine, melamine polyphosphate, and triguanamine, as well as (1) an aminotriazine sulfate compound such as guanylmelamine sulfate, melem sulfate, and melam sulfate, (2) a co-condensed product of phenols such as phenol, cresol, xylenol, butylphenol, and nonylphenol with melamines such as melamine, benzoguanamine, acetoguanamine, and formoguanamine, and formaldehyde, (3) a mixture of the co-condensed product (2) and phenol resins such as phenol formaldehyde condensate, and (4) the co-condensed product (2) or the mixture (3) further modified with tung oil, isomerized linseed oil, or the like.

Examples of the cyanuric acid compound include cyanuric acid, and melamine cyanurate.

The amount of the nitrogen-containing flame retardant blended is appropriately selected depending on the type of nitrogen-containing flame retardant, the other component of the resin composition, and a desired degree of flame retardancy. For example, the nitrogen-containing flame retardant is preferably blended in an amount of 0.05 to 10 parts by mass, and more preferably 0.1 parts by mass to 5 parts by mass, in 100 parts by mass of the resin composition in which the halogen free flame retardant, filler and other additives are all blended.

When the nitrogen-containing flame retardant is used, a metal hydroxide, a molybdenum compound, or the like may be used in combination.

As the silicone-based flame retardant, an organic compound containing a silicon atom can be used without particular limitation. Examples thereof include a silicone oil, a silicone rubber, and a silicone resin. The amount of the silicone-based flame retardant blended is appropriately selected depending on the type of the silicone-based flame retardant, the other component of the resin composition, and a desired degree of flame retardancy. For example, the silicone-based flame retardant is preferably blended in an amount of 0.05 to 20 parts by mass in 100 parts by mass of the resin composition in which the halogen free flame retardant, filler and other additives are all blended. When the silicone-based flame retardant is used, a molybdenum compound, alumina, or the like may be used in combination.

Examples of the inorganic flame retardant include a metal hydroxide, a metal oxide, a metal carbonate compound, a metal powder, a boron compound, and a low-melting-point glass.

Examples of the metal hydroxide include aluminum hydroxide, magnesium hydroxide, dolomite, hydrotalcite, calcium hydroxide, barium hydroxide, and zirconium hydroxide.

Examples of the metal oxide include zinc molybdate, molybdenum trioxide, zinc stannate, tin oxide, aluminum oxide, iron oxide, titanium oxide, manganese oxide, zirconium oxide, zinc oxide, molybdenum oxide, cobalt oxide, bismuth oxide, chromium oxide, nickel oxide, copper oxide, and tungsten oxide.

Examples of the metal carbonate compound include zinc carbonate, magnesium carbonate, calcium carbonate, barium carbonate, basic magnesium carbonate, aluminum carbonate, iron carbonate, cobalt carbonate, and titanium carbonate.

Examples of the metal powder include aluminum, iron, titanium, manganese, zinc, molybdenum, cobalt, bismuth, chromium, nickel, copper, tungsten, and tin.

Examples of the boron compound include zinc borate, zinc metaborate, barium metaborate, boric acid, and borax.

Examples of the low-melting-point glass include CEEPREE (Bokusui Brown Co., Ltd), hydrated glass SiO₂-MgO-H₂O, and PbO-B₂O₃-based, ZnO-P₂O₅-MgO-based, P₂O₅-B₂O₃-PbO-MgO-based, P-Sn-O-F-based, PbO-V₂O₅-TeO₂-based, Al₂O₃-H₂O-based, and lead borosilicate-based glass compounds.

The amount of the inorganic flame retardant blended is appropriately selected depending on the type of inorganic flame retardant, the other component of the resin composition, and a desired degree of flame retardancy. For example, the inorganic flame retardant is preferably blended in an amount of 0.05 parts by mass to 20 parts by mass, and more preferably 0.5 parts by mass to 15 parts by mass, in 100 parts by mass of the resin composition in which the halogen free flame retardant, filler and other additives are all blended.

Examples of the organic metal salt-based flame retardant include ferrocene, an acetylacetonate metal complex, an organic metal carbonyl compound, an organic cobalt salt compound, an organic sulfonic acid metal salt, and a compound in which a metal atom is ionically bonded or coordinately bonded to an aromatic compound or a heterocyclic compound.

The amount of the organic metal salt-based flame retardant blended is appropriately selected depending on the type of organic metal salt-based flame retardant, the other component of the resin composition, and a desired degree of flame retardancy. For example, the organic metal salt-based flame retardant is preferably blended in an amount of 0.005 parts by mass to 10 parts by mass in 100 parts by mass of the resin composition in which the halogen free flame retardant, filler and other additives are all blended.

The curable resin composition of the present invention may contain a filler. Examples of the filler include inorganic and organic fillers. Examples of the inorganic filler include inorganic fine particles.

Examples of the inorganic fine particles that are excellent in heat resistance include alumina, magnesia, titania, zirconia, silica (quartz, fumed silica, precipitated silica, anhydrous silica, fused silica, crystalline silica, ultra-fine amorphous silica, etc.). Examples of the inorganic fine particles that are excellent in thermal conductivity include boron nitride, aluminum nitride, aluminum oxide, titanium oxide, magnesium oxide, zinc oxide, silicon oxide, and diamond. Examples of the inorganic fine particles that are excellent in electrical conductivity include metal fillers and/or metal-coated fillers using metals or alloys (e.g., iron, copper, magnesium, aluminum, gold, silver, platinum, zinc, manganese, stainless steel, etc.), tin oxide, and indium oxide. Examples of the inorganic fine particles that are excellent in barrier properties include minerals such as mica, clay, kaolin, talc, zeolite, wollastonite, and smectite, potassium titanate, magnesium sulfate, sepiolite, zonolite, aluminum borate, calcium carbonate, titanium oxide, barium sulfate, zinc oxide, and magnesium hydroxide. Examples of the inorganic fine particles that have high refractive index include barium titanate, zirconia oxide, and titanium oxide. Examples of the inorganic fine particles that exhibit photocatalytic properties include photocatalytic metals such as titanium, cerium, zinc, copper, aluminum, tin, indium, phosphorus, carbon, sulfur, ruthenium, nickel, iron, cobalt, silver, molybdenum, strontium, chromium, barium, and lead, complexes of the metals, and oxides thereof. Examples of the inorganic fine particles that are excellent in abrasion resistance include metals such as silica, alumina, zirconia, and magnesium oxide, and complexes and oxides thereof. Examples of the inorganic fine particles that are excellent in insulating properties include silica. Examples of the inorganic fine particles that are excellent in shielding of ultraviolet light include titanium oxide and zinc oxide. These inorganic fine particles may be appropriately selected according to use application. The inorganic fine particles may be used alone, or a combination of a plurality of types thereof may be used. Since the inorganic fine particles have various properties other than the properties exemplified, the inorganic fine particles may be appropriately selected according to use application.

For example, when silica is used as the inorganic fine particles, publicly known silica fine particles such as powdered silica or colloidal silica can be used without particular limitation. Examples of commercially available powdered silica fine particles include AEROSIL 50 and 200 available from NIPPON AEROSIL CO., LTD., Sildex H31, H32, H51, H52, H121, and H122 available from Asahi Glass Co., Ltd., E220A and E220 available from Nippon Silica Industrial Co., Ltd., SYLYSIA470 available from FUJI SILYSIA CHEMICAL LTD., and SG flake available from Nippon Sheet Glass Co., Ltd.

Examples of commercially available colloidal silica include methanol silica sol, IPA-ST, MEK-ST, NBA-ST, XBA-ST, DMAC-ST, ST-UP, ST-OUP, ST-20, ST-40, ST-C, ST-N, ST-O, ST-50, and ST-OL available from Nissan Chemical Corporation.

Surface-modified silica fine particles may be used. Examples thereof include those surface-treated with a reactive silane coupling agent having a hydrophobic group and those modified with a compound having a (meth)acryloyl group. Examples of commercially available powdered silica modified with a compound having a (meth)acryloyl group include AEROSIL RM50 and R711 available from NIPPON AEROSIL CO., LTD. Examples of commercially available colloidal silica modified with a compound having a (meth)acryloyl group include MIBK-SD available from Nissan Chemical Corporation.

The shape of the silica fine particles is not particularly limited. Spherical, hollow, porous, rod-shaped, plate-shaped, fibrous, or irregularly shaped silica fine particles can be used. The primary particle size is preferably in the range of 5 to 200 nm.

As titanium oxide fine particles, not only an extender pigment but also ultraviolet light-responsive photocatalyst can be used. For example, anatase-type titanium oxide, rutile-type titanium oxide, brookite-type titanium oxide, or the like can be used. In addition, particles that are designed to respond to visible light by doping a crystal structure of titanium oxide with a heterologous element can be used. As an element with which titanium oxide is doped, an anionic element such as nitrogen, sulfur, carbon, fluorine, or phosphorus, or a cationic element such as chromium, iron, cobalt, or manganese is suitably used. As the state of titanium oxide, a powder, or a sol or a slurry in which titanium oxide is dispersed in an organic solvent or water can be used. Examples of commercially available powdered titanium oxide fine particles include AEROSIL P-25 available from NIPPON AEROSIL CO., LTD., and ATM-100 available from TAYCA CORPORATION. Examples of commercially available titanium oxide fine particles in a slurry state include TKD-701 available from TAYCA CORPORATION.

The curable resin composition of the present invention may further contain a fibrous matrix. The fibrous matrix is not limited, but those used for fiber-reinforced resins are preferred, and examples include inorganic and organic fibers.

Examples of the inorganic fibers include inorganic fibers such as carbon fiber, glass fiber, boron fiber, alumina fiber, and silicon carbide fiber, as well as carbon fiber, activated carbon fiber, graphite fiber, tungsten carbide fiber, silicon carbide fiber, ceramic fiber, natural fiber, mineral fiber such as basalt, boron nitride fiber, boron carbide fiber, and metallic fiber. Examples of the metallic fiber include aluminum fiber, copper fiber, brass fiber, stainless steel fiber, and steel fiber.

Examples of the organic fibers include synthetic fibers made of resin materials such as polybenzazole, aramid, PBO (polyparaphenylenebenzoxazole), polyphenylene sulfide, polyester, acrylic, polyamide, polyolefin, polyvinyl alcohol, and polyarylate, natural fibers such as cellulose, pulp, cotton, wool, and silk, and regenerated fibers such as protein, polypeptide, and alginic acid.

Among these, carbon fiber and glass fiber are preferred because of their wide range of industrial applications. Only one of these types may be used, or multiple types may be used simultaneously.

The fibrous matrix may be an aggregate of fibers, and the fibers may be continuous or discontinuous, and may be woven or non-woven. The fibrous matrix may be a fiber bundle in which fibers are aligned in one direction, or a sheet of fiber bundles. The fibrous matrix may have a three-dimensional shape in which the aggregate of fibers has a thickness.

In order to adjust the solid content and the viscosity of the resin composition, a dispersion medium may be used in the curable resin composition of the present invention. The dispersion medium is not limited as long as it is a liquid dispersion medium that does not impair the effects of the present invention. Examples thereof include various organic solvents and liquid organic polymers.

Examples of the organic solvent include ketones such as acetone, methyl ethyl ketone (MEK), and methyl isobutyl ketone (MIBK), cyclic ethers such as tetrahydrofuran (THF) and dioxolane, esters such as methyl acetate, ethyl acetate, and butyl acetate, aromatic compounds such as toluene and xylene, alcohols such as carbitol, cellosolve, methanol, isopropanol, butanol, and propylene glycol monomethyl ether. The organic solvents may be used alone or in combination. Among these, methyl ethyl ketone is preferred in terms of volatility during coating and solvent recovery.

The liquid organic polymer is a liquid organic polymer that does not contribute directly to a curing reaction. Examples thereof include an acrylic polymer (FLOWLEN WK-20: Kyoeisha Chemical Co., Ltd.), an amine salt of specialized modified phosphate (HIPLAAD ED-251: Kusumoto Chemicals, Ltd.), and a modified acrylic block copolymer (DISPERBYK2000: BYK).

The resin composition of the present invention may contain other additives. Examples include catalyst, polymerization initiator, inorganic pigment, organic pigment, extender pigment, clay mineral, wax, surfactant, stabilizer, flowing adjuster, coupling agent, dye, leveling agent, rheology control agent, UV absorber, antioxidant, flame retardant, plasticizer, and reactive diluent.

The resin composition of the present invention may be a curable resin composition that is a self-repairable composition or a composition for remolding materials.

The resin composition of the present invention may be cured to obtain a cured article. The curing can be performed at normal temperature or by heating. When thermal curing is performed, the resin composition may be cured in a single heating step or may be cured through a multi-step heating process.

The curable resin composition of the present invention can be cured with active energy rays. In this case, a photo-cationic polymerization initiator may be used as a polymerization initiator. Visible light, ultraviolet rays, X-rays, electron beams, and the like can be used as the active energy rays.

Examples of the photo-cationic polymerization initiator that can be used include aryl-sulfonium salts and aryl-iodonium salts. Specific examples include arylsulfonium hexafluorophosphate, arylsulfonium hexafluoroanthimonate, arylsulfonium tetrakis(pentafluoro)borate salt, and tri(alkylphenyl)sulfonium hexafluorophosphate. The photo-cationic polymerization initiator may be used alone, or two or more types thereof may be used in combination.

The curable resin composition of the present invention is obtained by uniformly mixing the components described above, and the mixing method is not limited. For example, the curable resin composition of the present invention can be prepared by uniformly mixing using a pot mill, a ball mill, a bead mill, a roll mill, a homogenizer, a super mill, a homo disper, a utility mixer, a Banbury mixer, a kneader, or the like.

The curable resin composition of the present invention is prepared by dissolving the amino group-containing compound of the present invention described above and the compound (I) having reactivity with the amino group-containing compound, and, if necessary, the curing agent, filler, fibrous matrix, dispersion medium, and resins other than the various compounds described above that can be used in combination, in the dispersion medium such as an organic solvent described above. After dissolution, the solvent is distilled off and the curable resin composition can be obtained by drying under reduced pressure in a vacuum oven or the like. The curable resin composition of the present invention may be a homogeneous mixture of the above constituent materials. In this case, it is preferable to mix the constituent materials uniformly in a mixer or the like. The ratio of each constituent material blended can be adjusted as appropriate according to desired properties such as mechanical strength, heat resistance, repairability, and remoldability of the cured article. In preparation of the curable resin composition, the order of mixing specific constituent materials is not particularly limited.

The cured article of the present invention is made by curing the compound (I) having reactivity with the amino group-containing compound by the amino group-containing compound of the present invention. As a curing method, a known method can be selected and employed as appropriate depending on the properties of the compound (I) having reactivity with the amino group-containing compound to be used.

The cured article of the present invention can maintain good mechanical strength by expressing moderate crosslink density as described above because it is cured by the amino group-containing compound of the present invention. When the cured article of the present invention is subjected to mechanical energy, such as scratches or external force, the reversible bond is broken, but since equilibrium shifts in the bonding direction, an adduct is formed again, which enables damage repair and remolding.

The structure of the resulting cured article can be determined by infrared absorption (IR) spectroscopy using Fourier transform infrared spectroscopy (FT-IR) or the like, elemental analysis, X-ray scattering, or the like.

The cured article according to one embodiment of the present invention can be obtained by using the amino group-containing compound of the present invention as one component of the curable resin composition, as described above. It is also possible to form the cured article while forming (synthesizing in situ) the amino group-containing compound in the process of curing, by using the dienophile intermediate, which is an intermediate of the amino group-containing compound, in combination with a compound capable of addition reaction by the Diels-Alder reaction.

For example, when a curing reaction is performed using the formula (1)', an anthracene having an amino group, and a compound (I) having reactivity with the amino group-containing compound as essential raw materials, the amino group-containing compound represented by the formula (1) can be obtained in the curing reaction process. In addition, a cured article can be obtained as the curing reaction proceeds. The anthracene having an amino group that can be used in this case is as defined above.

The curable resin composition of the present invention and the cured article produced from the curable resin composition are excellent in both heat resistance and repairability and have remoldability, and are useful for the following applications.

The curable resin cured article of the present invention can be laminated products with a substrate to form a laminate. As the substrate of the laminated product, an inorganic material such as metal or glass, an organic material such as plastic or wood, or the like can be used as appropriate depending on the application. The substrate can be in the form of a laminate, or may be a flat plate, a sheet, or may have a three-dimensional structure, or may have a three-dimensional shape. The substrate may be of any shape according to the purpose, for example, a shape having curvature on all or part of the surface. The hardness, thickness, and the like of the substrate are not limited. The substrate may be a multilayer laminate including a first substrate, a layer of the cured article of the curable resin composition of the present invention, and a second substrate in sequence. Since the curable resin composition of the present embodiment has excellent adhesiveness, it can be suitably used as an adhesive to bond the first substrate and the second substrate. The curable resin cured article of the present invention may be used as a substrate with which the cured article of the present invention may be further laminated.

In addition, the curable resin cured article of the present invention is particularly suitable for adhesion between different kinds of materials because of its ability to relax stress. For example, even in a laminate of different types of materials, such as a substrate of metal and/or metal oxide and a second substrate of a plastic layer, the adhesive strength is maintained due to the stress relaxation ability of the cured article of the present invention.

In the laminated product made by laminating the cured article of the present invention and the substrate, a layer containing the cured article may be formed by applying or molding directly onto the substrate, or a layer already molded may be laminated. In the case of directly applying, examples of the applying method include, but are not particularly limited, a spraying method, a spin coating method, a dip method, a roll-coating method, a blade coating method, a doctor roll method, a doctor blade method, a curtain coating method, a slit coating method, a screen printing method, and an inkjet method. In the case of directly molding, examples include in-mold molding, insert molding, vacuum molding, extrusion laminate molding, and press molding. When laminating the molded composition, an uncured or semi-cured composition layer may be laminated and then cured, or a layer containing a cured article in which the composition is fully cured may be laminated on the substrate. A precursor that can be a substrate may be applied to the cured article of the present invention and cured to form a laminate, or a precursor that can be a substrate or the composition of the present invention may be bonded in an uncured or semi-cured state and then cured. The precursor that can be a substrate is not limited, and examples include various curable resin compositions.

The cured article obtained using the curable resin composition of the present invention has particularly high adhesiveness to metals and/or metal oxides and can be used particularly well as a primer for metals. Examples of the metals include copper, aluminum, gold, silver, iron, platinum, chromium, nickel, tin, titanium, zinc, various alloys, and composite materials of these metals. Examples of the metal oxides include oxides and/or composite oxides of these metals. In particular, the cured article of the present invention has excellent adhesive strength to iron, copper, and aluminum, and can be used well as an adhesive for iron, copper, and aluminum.

The curable resin composition of the present invention can be suitably used as an adhesive for a structural member in the fields of automobiles, electric trains, civil engineering and construction, electronics, airplanes, and aerospace industry. For example, even when the adhesive is used in adhesion between different materials such as a metal and a non-metal, high adhesiveness can be maintained without being affected by a change in temperature environment, and peeling is unlikely to occur. The adhesive can be used as an adhesive for general office use, a medical adhesive, an adhesive for carbon fibers, or an adhesive for battery cells, modules or cases, in addition to the adhesive for a structural member. The adhesive can be used as an adhesive for bonding of an optical component, an adhesive for bonding of an optical disk, an adhesive for mounting of a printed wiring board, a die bonding adhesive, an adhesive for semiconductor such as an underfill material, and an adhesive for mounting such as an underfill material for BGA reinforcement, an anisotropic conductive film, and an anisotropic conductive paste.

When the curable resin composition of the present invention has a fibrous matrix and the fibrous matrix is a reinforcing fiber, the curable resin composition containing the fibrous matrix can be used as a fiber-reinforced resin. The fibrous matrix can be included in the composition by any method as long as the effects of the present invention are not impaired. For example, the fibrous matrix and the composition are composited by a method such as kneading, coating, impregnation, injection, or pressure bonding. These methods can be selected as appropriate depending on the form of fibers and the application of the fiber reinforced resin.

The fiber-reinforced resin may be molded by any method. When a plate-shaped product is produced, extrusion molding is common but flat pressing may be employed. In addition, extrusion molding, blow molding, compression molding, vacuum molding, injection molding, or the like can be used. When a film-shaped product is produced, melt extrusion as well as solution casting can be used. When melt molding is used, examples include film blown extrusion, casting, extrusion lamination, calendering, sheet molding, fiber molding, blow molding, injection molding, rotational molding, and coating molding. In the case of a resin that is cured with active energy rays, the cured article can be produced using various curing methods using active energy rays. In particular, when a thermosetting resin is used as the main component of a matrix resin, exemplary molding methods include a method in which a molding material pre-impregnated is pressed and heated by a press or an autoclave. Other methods include resin transfer molding (RTM), vacuum assist resin transfer molding (VaRTM), laminate molding, and hand layup molding.

The cured article of the curable resin composition of the present invention has good heat resistance and good repairability, and has remoldability, and thus can be used as a molding material for large cases, motor housings, cast-molded materials inside cases, gears and pulleys, and the like. These may be cured articles of the resin alone or may be cured articles reinforced with fibers such as glass chips.

The fiber-reinforced resin can form a state called uncured or semi-cured prepreg. After a product in a prepreg state is distributed, final curing may be performed to form a cured article. When a laminated product is formed, it is preferable to form a prepreg and then laminate other layers before final curing to form a laminate in which each layer adheres to each other. The mass ratio of the composition and the fibrous matrix used in this case is not particularly limited. It is preferable that the prepreg be prepared such that the ratio of the resin in the prepreg is generally 20 to 60% by mass.

The cured article of the present invention has good heat resistance and good repairability, and has remoldability, and can be used as a heat resistant material and electronic material. In particular, the cured article of the present invention is suitable for semiconductor sealing materials, circuit boards, build-up films, and build-up substrates, as well as adhesives and resist materials. It is also suitable for a matrix resin for fiber-reinforced resins, and is particularly suitable as a prepreg with high heat resistance. Heat-resistant members and electronic members thus obtained can be suitably used in various applications. Examples of the applications include industrial machine parts, general machine parts, parts for automobiles, railroads, vehicles, and the like, space and aviation-related parts, electronic and electrical parts, construction materials, containers and packaging members, household goods, sports and leisure goods, and housing parts for wind power generation. Examples of the applications are not limited to those listed above.

In particular, the cured article can be suitably used as an adhesive for a structural member in the fields of automobiles, electric trains, civil engineering and construction, electronics, airplanes, and aerospace industry by using characteristics including excellent flexibility of the cured article. For example, even when an adhesive of the present invention is used in adhesion between different materials such as a metal and a non-metal, high adhesiveness can be maintained without being affected by a change in temperature environment, and peeling is unlikely to occur. The adhesive of the present invention can be used as an adhesive for general office use, a medical adhesive, an adhesive for carbon fibers, or an adhesive for battery cells, modules, and cases, in addition to the adhesive for a structural member. Examples of the adhesive of the present invention include an adhesive for bonding of an optical component, an adhesive for bonding of an optical disk, an adhesive for mounting of a printed wiring board, a die bonding adhesive, an adhesive for semiconductor such as an underfill material, and an adhesive for mounting such as an underfill material for BGA reinforcement, an anisotropic conductive film, and an anisotropic conductive paste.

Examples of typical products will be described below.

### 1. Semiconductor Sealing Material

Examples of a method for preparing a semiconductor sealing material from the resin composition of the present invention include a method in which the resin composition, and a compounding agent such as the curing accelerator and the inorganic filler are sufficiently melt-mixed using an extruder, a kneader, a roll, or the like, if necessary, until the components are homogeneous. In this case, as the inorganic filler, molten silica is generally used. When the molten silica is used as a high thermal conductive semiconductor sealing material for a power transistor or a power IC, filling of crystalline silica, alumina, or silicon nitride having higher thermal conductivity than molten silica may be increased, or molten silica, crystalline silica, alumina, silicon nitride, or the like may be used. The inorganic filler is used preferably at a filling rate of 30 to 95% by mass per 100 parts by mass of the curable resin composition. In particular, in terms of improving flame retardancy, moisture resistance, and resistance to solder cracking, and decreasing a linear expansion coefficient, the filling rate is more preferably 70 parts by mass or more, and even more preferably 80 parts by mass or more.

### 2. Semiconductor Device

Examples of semiconductor packaging for preparing a semiconductor device from the curable resin composition of the present invention include a method in which the semiconductor sealing material is cast or molded with a transfer molding machine, an injection molding machine, or the like and then heated at 50 to 250°C for 2 to 10 hours.

### 3. Printed Circuit Board

Examples of a method for preparing a printed circuit board from the composition of the present invention include a method in which the prepreg is layered by an ordinary method, copper foil is appropriately overlaid, and they are thermally compression-bonded under a pressure of 1 to 10 MPa at 170 to 300°C for 10 minutes to 3 hours.

### 4. Flexible Substrate

Examples of a method for producing a flexible substrate from the crosslinkable resin composition of the present invention include a production method including three steps described below.

A first step is a step of applying a crosslinkable resin composition containing a resin component, an organic solvent, and the like to an electrically insulating film using a coater such as a reverse roll coater or a comma coater.

A second step is a step of heating the electrically insulating film having the applied crosslinkable resin composition at 60 to 170°C for 1 to 15 minutes using a heater and volatilizing the solvent from the electrically insulating film, to obtain the crosslinkable resin composition in a B-stage state.

A third step is a step of thermocompression-bonding the electrically insulating film having the crosslinkable resin composition in the B-stage state with metal foil through an adhesive using a heating roll or the like (a thermocompression-bonding pressure is preferably 2 to 200 N/cm, and a thermocompression-bonding temperature is preferably 40 to 200°C).

If sufficient adhesion performance is achieved through the three steps, the operation may be completed. If complete adhesion performance is required, post-curing under conditions of 100 to 200°C and 1 to 24 hours is preferably further performed. The thickness of a finally cured layer of the resin composition is preferably within the range of 5 to 100 µm.

### 5. Build-Up Substrate

A method for preparing a build-up substrate from the composition of the present invention includes, for example, the following steps. First, the above composition containing a rubber, a filler, and the like as appropriate is applied to a circuit board having circuitry using spray coating, curtain coating, or the like and then cured (step 1). Then, after drilling the circuit board to form a predetermined through hole section as required, the circuit board is treated with a roughening agent, and the surface is washed with hot water to form irregularities and then plated with a metal such as copper (step 2). These operations are repeated sequentially as desired so that a resin insulating layer and a conductor layer with a predetermined circuit pattern are alternately built up (step 3). The drilling for the through hole section is performed after formation of an outermost resin insulating layer. The build-up substrate of the present invention can also be produced by thermally compression-bonding a copper foil with a resin that is obtained by semi-curing the resin composition on the copper foil on a wiring substrate having a circuit at 170 to 300°C without forming a roughened surface and a plating treatment.

### 6. Build-Up Film

As a method for preparing a build-up film from the composition of the present invention, the composition is applied to a surface of a support film (Y) as a substrate, and an organic solvent is dried by heating, blowing hot air, or the like, to form a composition layer (X).

Preferred examples of the organic solvent used herein include ketones such as acetone, methyl ethyl ketone, and cyclohexanone; acetic esters such as ethyl acetate, butyl acetate, cellosolve acetate, propylene glycol monomethyl ether acetate, and carbitol acetate; carbitols such as cellosolve and butyl carbitol; aromatic hydrocarbons such as toluene and xylene, dimethylformamide, dimethylacetamide, and N-methylpyrrolidone. The organic solvent is preferably used at such a ratio that the nonvolatile content is 30 to 60% by mass.

In general, the thickness of the formed layer (X) is equal to or more than the thickness of the conductor layer. Since the thickness of the conductor layer in the circuit board is generally within the range of 5 to 70 µm, the thickness of the resin composition layer is preferably within the range of 10 to 100 µm. The composition layer (X) in the present invention may be protected by a protective film described below. Protection by the protective film can prevent a surface of the resin composition layer from suffering attachment of contaminants or the like or from being scratched.

Examples of the support film and the protective film include polyolefins such as a polyethylene, a polypropylene, and a polyvinyl chloride, polyesters such as a polyethylene terephthalate (hereinafter also referred to as "PET") and a polyethylene naphthalate, polycarbonates, polyimides, a release paper, and metal foils such as a copper foil and an aluminum foil. The support film and the protective film may be subjected to matting and corona treatment as well as releasing treatment. The thickness of the support film is not particularly limited, and the support film generally having a thickness of the range of 10 to 150 µm, and preferably 25 to 50 µm is used. The thickness of the protective film is preferably 1 to 40 µm.

The support film (Y) is peeled after lamination on the circuit board or formation of an insulating layer by heating and curing. When the curable resin composition layer that constitutes the build-up film is heated and cured and the support film (Y) is then peeled, attachment of contaminants or the like at a curing step can be prevented. When the support film is peeled after curing, the support film is generally subjected to a releasing treatment in advance.

A multilayered printed wiring board can also be produced by using the build-up film obtained as described above. For example, if the layer (X) is protected by the protective film, these are peeled, and then the layer (X) is laminated directly in contact with a surface or both surfaces of the circuit board, for example, by a vacuum lamination method. The lamination method may be in a batch mode or a continuous mode with rolls. Before lamination, the build-up film and the circuit board may be heated (preheated), if necessary. The lamination is preferably performed under a lamination condition of a compression-bonding temperature (lamination temperature) of 70 to 140°C, a compression-bonding pressure of 1 to 11 kgf/cm² (9.8 × 10⁴ to 107.9 × 10⁴ N/m²), and a reduced pressure of air pressure of 20 mm Hg (26.7 hPa) or less.

### 7. Conductive paste

Examples of a method for preparing conductive paste from the composition of the present invention include a method in which conductive particles are dispersed in the composition. The conductive paste can be a paste resin composition for circuit connection or an anisotropic conductive adhesive, depending on the type of conductive particles used.

### Examples

The present invention will now be specifically described using examples and comparative examples. Unless otherwise specified, "part(s)" and "%" in the following description are based on mass. The present invention is not limited to these examples.

### "Thin-Layer Chromatography"

Silica gel plates purchased from AS ONE CORPORATION were used.

Product No. 4-2547-01, Model No. G2020, 200 nm × 200 nm, 250 µm thickness, measurement conditions (developing solvent): hexane/dichloromethane = 1/3

### "Silica Gel Column Chromatography"

Silica gel for chromatography "CHROMATOREX (registered trademark) PSQ60B" from FUJI SILYSIA CHEMICAL LTD. was used.

Developing solvent: dichloromethane/ethyl acetate = 3/1

¹H and ¹³C-NMR, FD-MS spectra, and GPC were measured under the following conditions.

¹H-NMR: ECA 500 from JEOL RESONANCE
Magnetic field intensity: 500 MHz
Integration count: 16 times
Solvent : Dichloromethane-d2

¹³C-NMR: "JNM-ECA600" from JEOL RESONANCE
Magnetic field intensity: 150 MHz
Integration count: 320 times
Solvent : DMSO-d₆
Sample concentration: 30% by mass

FD-MS: "JMS-T100GC AccuTOF" from JEOL Ltd.
Measurement range: m/z = 50.00 to 2000.00
Change rate: 25.6 mA/min
Final electric current: 40 mA
Cathode voltage: -10 kV

GPC: "HLC-8320GPC" from Tosoh Corporation
Columns: "TSK-GEL G2000HXL" + "TSK-GEL G3000HXL" + "TSK-GEL G4000HXL" from Tosoh Corporation
Detector: RI (differential refractometer)
Measuring conditions: 40°C
Mobile phase: tetrahydrofuran
Flow rate: 1 ml/min
Standard: "PStQuick A", "PStQuick B", "PStQuick E", "PStQuick F" from Tosoh Corporation

The epoxy equivalent weight of the synthesized epoxy resin was measured in accordance with JIS K7236, and the epoxy equivalent weight (g/eq) was calculated.

Examples of the method for calculating the number of repeating units include GPC molecular weight measurement or calculation from the results of various appropriate instrumental analyses such as FD-MS and NMR.

### <Synthesis Example 1>

### "Synthesis of 2-Aminoanthracene (AA)"

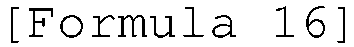

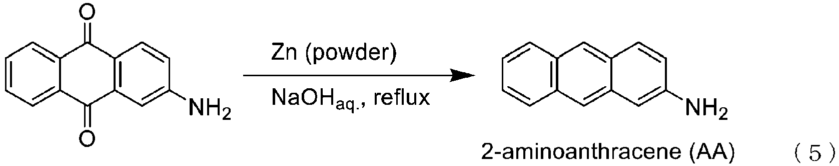

2-Aminoanthracene (AA) was synthesized according to the scheme represented by the formula (5). In a 500-mL three-necked flask, 10.0 g (44.8 mmol) of 2-aminoanthraquinone (FUJIFILM Wako Pure Chemical Corporation), 8.42 g (0.129 mol) of Zn (powder, Tokyo Chemical Industry Co., Ltd.), and 105 mL of 10% sodium hydroxide solution were added and stirred (room temperature /30 min). Then, 5.26 g (80.4 mmol) of Zn (powder) was added, then the temperature was raised to 100°C using an oil bath, and the mixture was stirred for 30 minutes. In addition, 5.26 g (80.4 mmol) of Zn (powder) was added, and the mixture was refluxed at an oil bath temperature of 115°C for 24 hours. After confirming the disappearance of the raw materials using thin-layer chromatography, the solid phase was cooled to room temperature and collected by suction filtration. The solid phase was washed with pure water and then dried under reduced pressure at 60°C overnight. After 1 L of acetone was added to the resulting solid, the acetone-insoluble portion was removed by suction filtration, and the soluble portion was collected. The acetone in the soluble portion was removed under reduced pressure and the residue was dried under reduced pressure (room temperature/overnight). The resulting solid was purified by recrystallization (solvent: toluene) to obtain 4.87 g of 2-aminoanthracene (AA) (green solid) (yield 56%).

### <Example 1>

### "Synthesis of Amino Group-Containing Compound (ABA) of Present Embodiment"

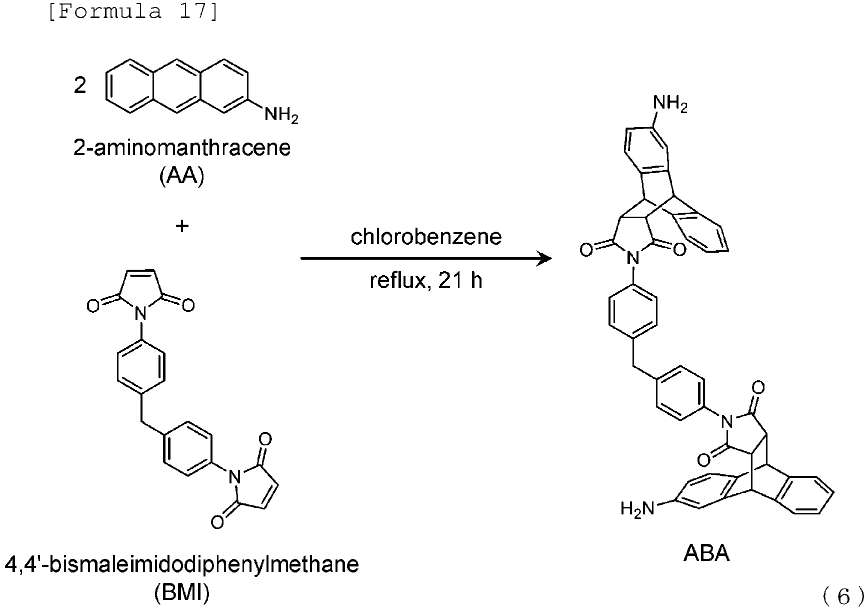

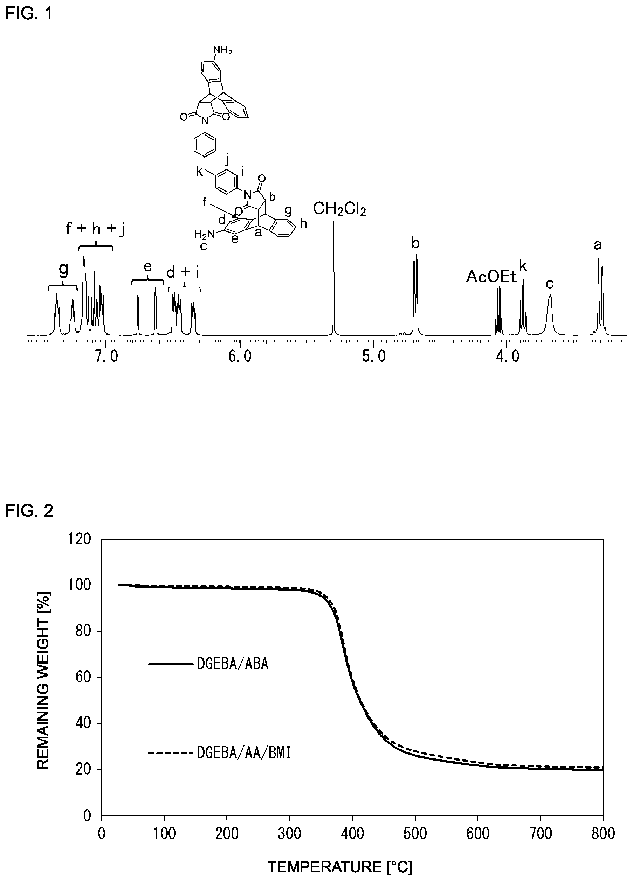

An amino group-containing compound ABA of the present embodiment with a Diels-Alder reaction unit was synthesized according to the scheme represented by the formula (6). In a 300-mL three-necked flask, 5.00 g (25.87 mmol, 2.2 eq.) of 2-aminoanthracene (AA) obtained by the method of Synthesis Example 1 above and 4.21 g (11.75 mmol) of 4,4'-bismaleimidodiphenylmethane (Tokyo Chemical Industry Co., Ltd.) were added. After the system was purged with argon (Ar), 125 mL of chlorobenzene (FUJIFILM Wako Pure Chemical Corporation) (degassed by Ar bubbling) was added. The resulting solution was heated to reflux for 21 hours, followed by solvent removal and drying under reduced pressure (70°C/overnight) to obtain a crude product. The crude product was purified by silica gel column chromatography (developing solvent: dichloromethane/ethyl acetate = 3/1) to obtain 8.49 g of a light orange solid (yield 97.0%). The ¹H-NMR spectrum of the light orange solid, measured using the above ¹H-NMR measurement method, is illustrated in FIG. 1. According to FIG. 1, peaks at 3.30 ppm and 4.69 ppm, which were derived from the bond formed between AA and BMI by the Diels-Alder reaction, confirmed the formation of the target compound (ABA). (D-1)

### <Example 2>

A reaction was carried out in the same manner as in Example 1 except that 4.21 g of 4,4'-bismaleimidodiphenylmethane in Example 1 was replaced by 3.74 g of 1,6'-bismaleimide-(2,2,4-trimethyl)hexane (BMI-THM from Daiwakasei Industry Co., LTD.) to obtain 8.04 g of a Diels-Alder reaction product (D-2). (yield 97.0%)

### <Synthesis Example 2>

A reaction was carried out in the same manner as in Synthesis Example 1 except that 10.0 g of 2-aminoanthraquinone in Synthesis Example 1 was replaced by 10.0 g of 1-aminoanthraquinone to obtain 6.72 g of 1-aminoanthracene. (yield 78.0%)

### <Example 3>

A reaction was carried out in the same manner as in Example 1 except that 5.00 g of 2-aminoanthracene in Example 1 was replaced by 5.00 g of 1-aminoanthracene obtained by the method of Synthesis Example 2 to obtain 7.79 g of a Diels-Alder reaction product (D-3). (yield 89.0%)

### <Synthesis Example 3>

N-(4-aminophenyl)maleimide (APM) was prepared. Specifically, 5.35 g (49.5 mmol) of p-phenylenediamine and 97 mL of tetrahydrofuran (THF) were added to a 300-mL eggplant flask, and 4.85 g (49.5 mmol) of maleic anhydride dissolved in 36 mL of THF was added dropwise over 1 hour at room temperature while stirring. After completion of adding dropwise, stirring (r.t./12h) was carried out, and the precipitate formed by suction filtration was collected and dried under reduced pressure (r.t./12h) to obtain 9.49 g of APMA (yellow solid).

Then, 9.47 g (45.9 mmol) of APMA was added to a 200-mL two-necked flask. After the system was purged with argon, 36 mL of N,N-dimethylformamide (DMF) and 9.60 mL (68.9 mmol) of triethylamine (NEt₃) were added, and the mixture was stirred and dissolved at room temperature. The flask was ice-cooled, and 10.6 mL (46.0 mmol) of di-tert-butyl bicarbonate (Boc₂O) was added and stirred for 15 minutes, followed by stirring for 17 hours at room temperature. The resulting solution was put into 257 mL of pure water (0°C) and then the pH was adjusted to 4.5 to 5.5 with dilute hydrochloric acid to form a precipitate. The precipitate was collected by suction filtration and dried under reduced pressure (70°C/12 h) to obtain 13.5 g of light yellow solid.

This light yellow solid was added in an amount of 13.2 g to a 1000-mL eggplant flask with 133 mL of acetic anhydride (Ac₂O), the temperature was raised to 95°C, and 3.88 g (47.3 mmol) of sodium acetate (AcONa) was added and stirred (95°C/2 h). The light yellow solid that precipitated by standing at room temperature for 12 hours after completion of stirring was collected by adding 800 mL of pure water and stirring (r.t./10 min), followed by suction filtration and washing with saturated sodium hydrogen carbonate solution and pure water. The resulting solid was dissolved in dichloromethane and washed with pure water and saturated brine, followed by drying with sodium sulfate, filtration, solvent removal, and drying under reduced pressure (r.t./12 h) to obtain 10.2 g of Boc-APM (light yellow solid).

Then, 56 mL of dichloromethane (CH₂Cl₂) was added to a 500-mL eggplant flask containing 10.1 g (35.0 mmol) of Boc-APM, and 68.5 g (0.601 mol) of trifluoroacetic acid (CF₃COOH) was added under ice bath, followed by stirring (r.t./2 h). After the solvent was removed after completion of the reaction, the residue was dissolved in pure water, and extraction with dichloromethane/saturated sodium hydrogen carbonate solution was performed three times. The resulting organic layer was washed with pure water and saturated brine, followed by drying with sodium sulfate, filtration, solvent removal, and drying under reduced pressure (r.t./12 h) to obtain a crude product of APM (5.84 g). The crude product was purified by silica gel column chromatography (eluent: chloroform/ethyl acetate = 20/1) to obtain 4.66 g of APM (red solid). The yield was 52% in four steps.

### <Synthesis Example 4>

A reaction was carried out in the same manner as in Example 1 except that 4.21 g of 4,4'-bismaleimidodiphenylmethane in Example 1 was replaced by 5.36 g of APM obtained by the method of Synthesis Example 3 to obtain 9.57 g of a Diels-Alder reaction product (D-4).

### <Synthesis Example 5>

In a flask equipped with a thermometer and a stirrer, 210 g (0.5 mol) of a diglycidyl ether of 1,12-dodecanediol (available from Yokkaichi Chemical Company Limited: epoxy equivalent 210 g/eq) and 119.7 g (0.53 mol) of bisphenol A (hydroxyl equivalent 114 g/eq) were charged. After the temperature was raised to 140°C for 30 minutes, 3.2 g of 20% sodium hydroxide solution was charged. The temperature was then raised to 150°C for 30 minutes, and a reaction was allowed to further proceed at 150°C for 16 hours. A neutralizing amount of sodium phosphate was then added to obtain 320 g of a hydroxy compound (Ph-1).

### <Synthesis Example 6>

In a flask equipped with a thermometer, a dropping funnel, a condenser, and a stirrer, 200 g of the hydroxy compound Ph-1 obtained in Synthesis Example 4, 437 g (4.72 mol) of epichlorohydrin, and 118 g of n-butanol were charged and allowed to dissolve under nitrogen gas purge. The temperature was raised to 65°C, and the pressure was reduced to a pressure in which azeotropy occurred. 6.66 g (0.08 mol) of 49% sodium hydroxide solution was added dropwise over 5 hours. Subsequently, stirring was continued under the same condition for 0.5 hour. During the reaction, the distillate that was distilled out by azeotropy was separated with a Dean Stark trap, and a water layer was removed while an oil layer was returned to the reaction system. The unreacted epichlorohydrin was then removed by vacuum distillation. To the resultant crude epoxy resin, 150 g of methyl isobutyl ketone and 150 g of n-butanol were added and allowed to dissolve. To this solution, 10 g of 10% sodium hydroxide solution was added, and a reaction was allowed to proceed at 80°C for 2 hours. The resultant was then washed with 50 g of water three times until the pH of the washing liquid was neutral. Subsequently, the system was dehydrated by azeotropy, microfiltration was performed, and the solvent was removed under reduced pressure, to obtain 190 g of an epoxy compound Ep-1. The obtained epoxy compound Ep-1 had an epoxy equivalent weight of 2320 g/eq.

### <Example 4>

### "Preparation of Epoxy Resin Cured Article Using Amino Group-Containing Compound (ABA) of Present Embodiment as Curing Agent"

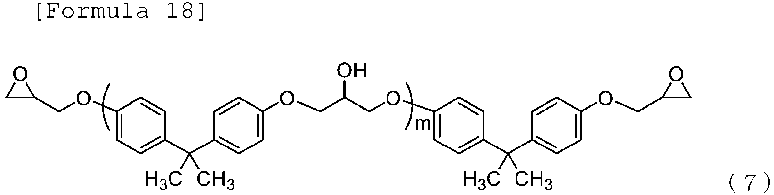

In a 300-mL eggplant flask, 2.00 g of the epoxy resin (DGEBA) represented by the formula (7) (EEW = 190 g/eq.), 1.96 g of ABA (active hydrogen equivalent = 186.21 g/eq.), and acetone were added and stirred (room temperature/30 min). The mixture was then concentrated by evaporation and dried under reduced pressure (room temperature/overnight) to obtain a light pink solid. Then, about half of the light pink solid was put into a silicone casting mold and melted in a vacuum oven at 130°C for 5 minutes. The remaining light pink solid was then put into the silicone casting mold and melted in a vacuum oven at 130°C for 5 minutes. Then, after degassing (130°C/20 min), heating and curing was performed at 140°C for 1 hour, 180°C for 2 hours, 200°C for 3 hours, and 220°C for 3 hours, in this order, to obtain a brown transparent cured article (DGEBA/ABA).

### <Example 5>

### "Preparation of Epoxy Resin Cured Article by Mixing AA/BMI/DGEBA"

In a 300-mL eggplant flask, 5.00 g of the epoxy resin (DGEBA) represented by the formula (7) (EEW = 190 g/eq.), 2.54 g of AA (active hydrogen equivalent = 96.63 g/eq.) (13.14 mmol), 2.36 g (6.586 mmol) of BMI, and acetone were added and stirred (room temperature/30 min). The mixture was then concentrated by evaporation and dried under reduced pressure (room temperature/overnight) to obtain a green viscous solid. The green viscous solid was then put into a silicone casting mold and melted in a vacuum oven at 130°C for 5 minutes. Then, after degassing (130°C/10 min), heating and curing was performed at 140°C for 1 hour, 180°C for 2 hours, 200°C for 3 hours, and 220°C for 3 hours, in this order, to obtain a brown transparent cured article (DGEBA/AA/BMI) .

### <Comparative Example 1>

In a 300-mL eggplant flask, 2.20 g of the epoxy resin (DGEBA) represented by the formula (7) (EEW = 190 g/eq.), 0.57 g of 4,4'-diaminodiphenylmethane (DDM) (active hydrogen equivalent = = 49.6 g/eq.), and acetone were added and stirred (room temperature/30 min). The mixture was then concentrated by evaporation and dried under reduced pressure (room temperature/4 hours) to obtain a curable resin composition. The curable resin composition was then put into a silicone casting mold and melted at 100°C in a vacuum oven. Then, after degassing (100°C/15 min), heating and curing was performed at 100°C for 1 hour, 160°C for 2 hours, 180°C for 3 hours, and 200°C for 3 hours, in this order, to obtain a yellow transparent cured article (DGEBA/DDM).

### <Example 6>

### [Evaluation of Heat Resistance of Cured Article]

Thermogravimetric analysis (TGA) of the epoxy resin cured articles "DGEBA/ABA" and "DGEBA/AA/BMI" obtained in Examples 4 and 5, respectively, was performed by TGA-50 from Shimadzu Corporation. The heating rate was 10°C/min, and the measurements were performed in a N₂ gas atmosphere. The TGA results of the epoxy resin cured articles are illustrated in FIG. 2. Both epoxy resin cured articles had a 5% weight loss temperature (Td5) above 350°C (DGEBA/ABA: 351°C, DGEBA/AA/BMI: 359°C), indicating that they have excellent chemical heat resistance. Furthermore, the residual carbon ratio at 800°C is as high as around 20% (DGEBA/ABA: 19.7%, DGEBA/AA/BMI: 20.9%), suggesting excellent flame retardancy.

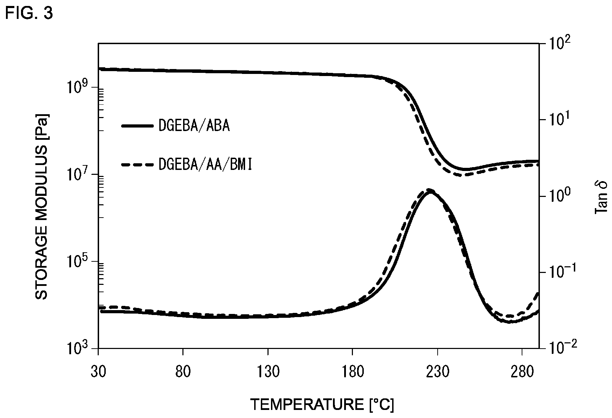

The glass transition temperatures (Tg) of the epoxy resin cured articles "DGEBA/ABA" and "DGEBA/AA/BMI" obtained in Examples 4 and 5, respectively, were measured by performing dynamic mechanical analysis (DMA) using DMS6100 from SII Nano Technology Inc. The measurements were performed at a heating rate of 5°C/min and a frequency of 1.0 Hz. The DMA results of the epoxy resin cured articles are illustrated in FIG. 3. Both epoxy resin cured articles had Tg above 200°C (DGEBA/ABA: 226°C, DGEBA/AA/BMI: 224°C), indicating that they have excellent physical heat resistance.

### <Comparative Example 2>

Thermogravimetric analysis (TGA) of the epoxy resin cured article "DGEBA/DDM" obtained in Comparative Example 1 was performed in the same manner as in Example 6. The residual carbon ratio at 800°C was 16.1%.

The glass transition temperature (Tg) was measured by performing dynamic mechanical analysis (DMA). Tg was 173°C.

### <Examples 7 to 13, Comparative Example 3>

### "Preparation of Composition and Cured Article"

In a 300-mL eggplant flask, each compound and acetone were added in accordance with the formulation in Table 1 (the numbers in the table are based on mass) and stirred (room temperature/30 min). The mixture was then concentrated by evaporation and dried under reduced pressure (room temperature/overnight) to obtain a curable resin composition. Next, each curable resin composition was heated and cured under predetermined conditions in a vacuum press to obtain a cured article with a thickness of 0.5 mm.

### <Comparative Example 4>

A curable resin composition was obtained by uniformly mixing each compound with a mixer ("Awatori Neritaro ARV-200" from THINKY CORPORATION) in accordance with the formulation in Table 1 (the numbers in the table are based on mass). The curable resin composition was sandwiched between aluminum mirror-finished plates ("JIS H 4000 A1050P" from Engineering Test Service Co., Ltd.) with a silicon tube as a spacer, and heated and cured under predetermined conditions to obtain a cured article with a thickness of 0.5 mm.

### <Comparative Example 5>

A curable resin composition was obtained by melt-kneading each compound in accordance with the formulation in Table 1 (the numbers in the table are based on mass). The curable resin composition was cured in a vacuum press at 150°C for 10 minutes, followed by after-curing at 175°C for 5 hours to obtain a cured article with a thickness of 0.5 mm.

### <Visual Observation Before and After Remolding>

The prepared cured article was frozen and crushed. 0.2 g of the crushed cured article was placed in a 10 mm square, 0.5 mm thick mold and vacuum-pressed under predetermined conditions. The appearance of the obtained cured article was visually observed. The criteria are as follows. The results are listed in Table 1.
A: Seams disappeared and the cured article became integrated.
B: Seams were partially visible, but the cured article became integrated.
C: Had solid shape and fell apart when light force was applied.

### <Tensile Test Before and After Remolding>

The produced cured article was cut out into a dumbbell shape (JIS K 7161-2-1BA) with a die cutter, and this was used as a specimen. Using a tensile tester ("Autograph AG-IS" from Shimadzu Corporation), breaking stress and tensile elongation (test speed: 2 mm/min) at 23°C in a measurement environment were determined in accordance with JIS K 7162-2. The cured article frozen and crushed was vacuum-pressed under predetermined conditions, and the produced cured article was cut into a dumbbell shape with a die cutter and evaluated by a tensile test in the same way.

The recovery rate (%) was calculated for the obtained breaking stress and tensile elongation based on the formula (value after recovery/initial value) × 100%.

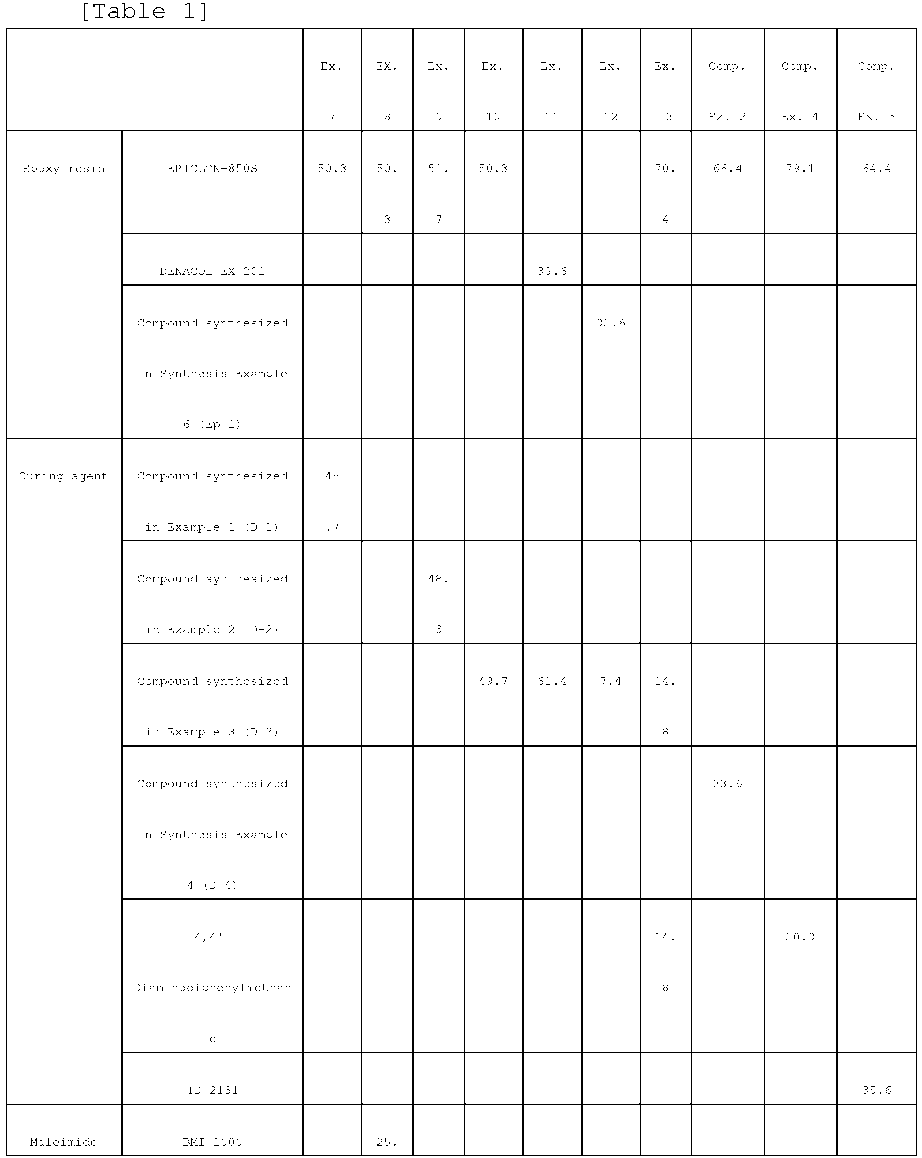

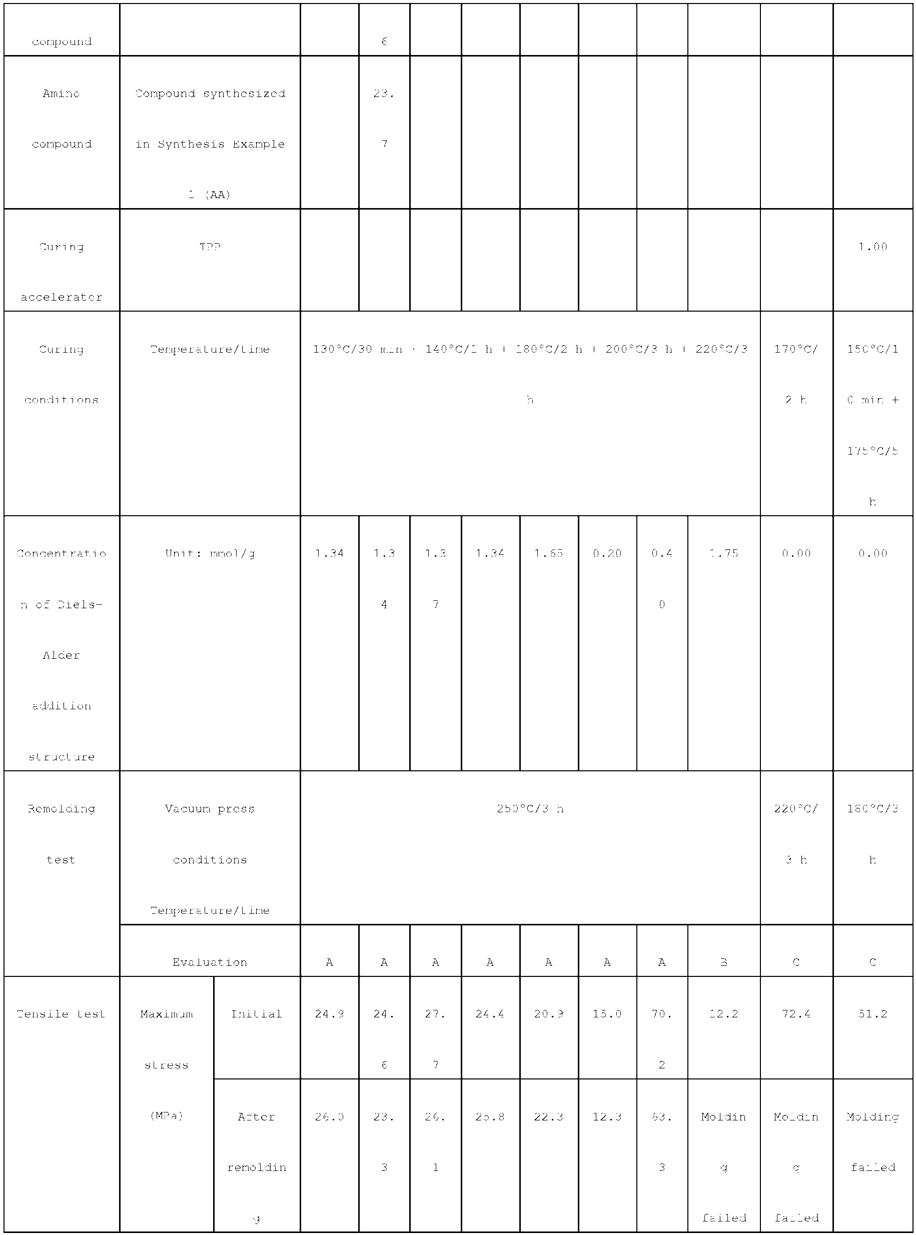

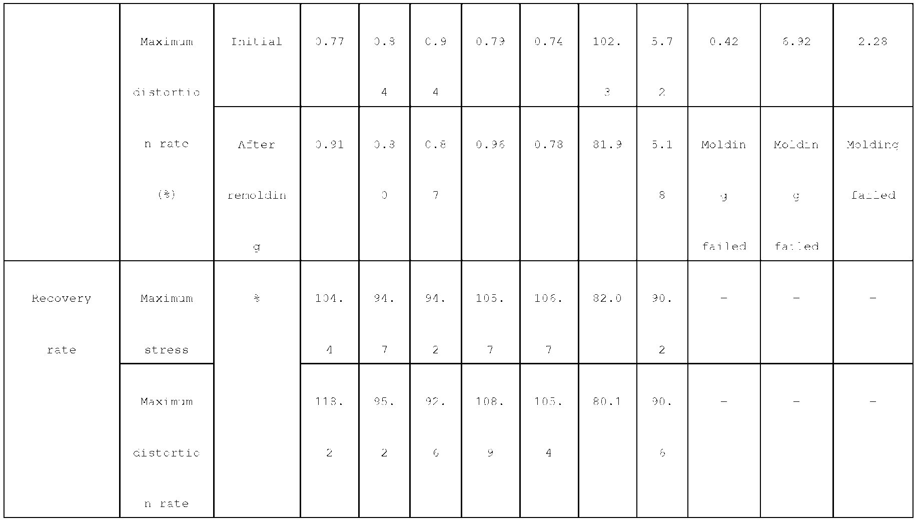

The components listed in the table are as follows.
EPICLON-850S: bisphenol A liquid epoxy resin (from DIC Corporation, epoxy equivalent 188 g/eq)
DENACOL EX-201: (from Nagase ChemteX, epoxy equivalent 117 g/eq)
TD-2131: phenol novolac phenol resin (from DIC Corporation, hydroxyl equivalent 104 g/eq)
4,4'-Diaminodiphenylmethane (from KANTO CHEMICAL CO., INC.)
TPP: triphenylphosphine (from Tokyo Chemical Industry Co., Ltd.)

## Claims

1. An amino group-containing compound represented by the following general formula (1) and having a molecular weight of less than 1000: 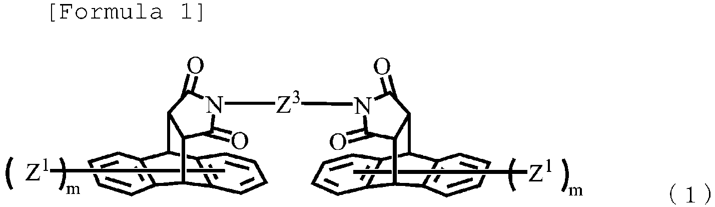
wherein m is an integer of 1 to 10;
Z¹'s are each independently a hydrogen atom, an amino group, a halogen atom, an alkoxy group, an aralkyloxy group, an aryloxy group, a nitro group, an amide group, an alkyloxycarbonyl group, an aryloxycarbonyl group, a cyano group, an alkyl group, a cycloalkyl group, an aralkyl group, or an aryl group, and at least one of Z¹'s is an amino group or a group having an amino group as a substituent; and
Z³ is any of the structures represented by the following formulae (2), 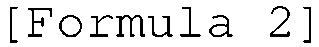 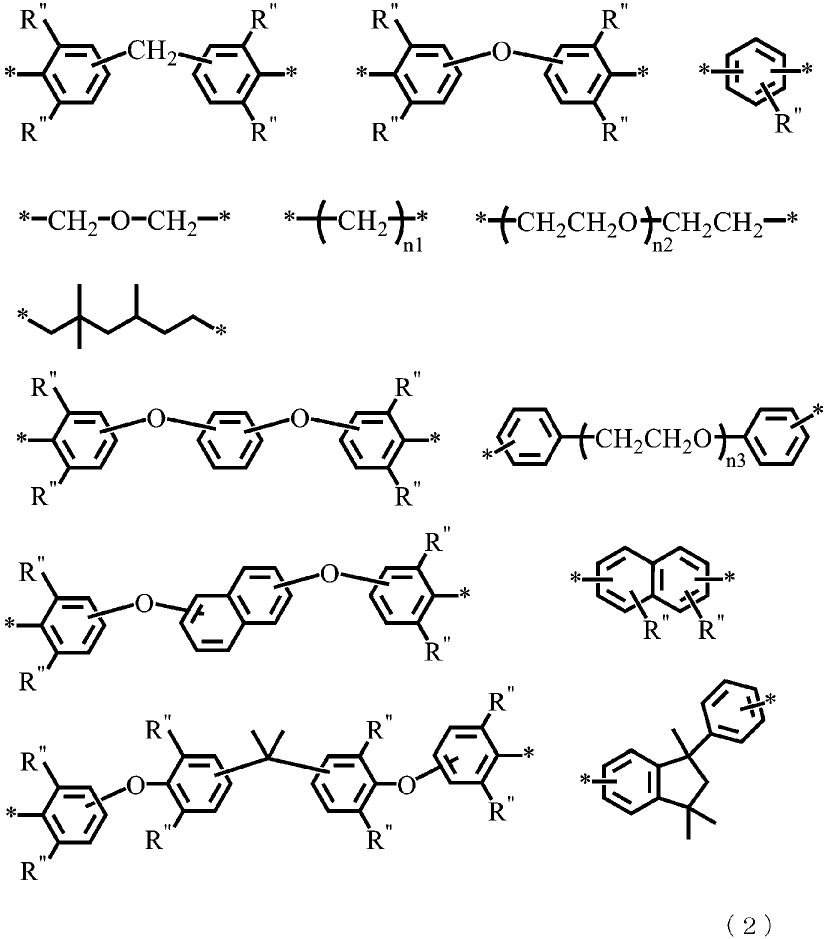
wherein each R'' is independently a hydrogen atom, a methyl group, or an ethyl group, n1 is an integer of 1 to 30, n2 is an average number of repetitions and is 0.5 to 8, n3 is an average number of repetitions and is 0.5 to 6, and * represents a bonding point.

2. A curable resin composition comprising, as essential components, the amino group-containing compound according to claim 1 and a compound (I) having reactivity with the amino group-containing compound.

3. The curable resin composition according to claim 2, wherein a concentration of reversible bonds in the amino group-containing compound to a total mass of curable components in the curable resin composition is 0.10 mmol/g or more.

4. The curable resin composition according to claim 2, wherein the compound (I) having reactivity with the amino group-containing compound is an epoxy resin.

5. The curable resin composition according to claim 4, further comprising a curing agent for epoxy resins other than the amino group-containing compound.

6. The curable resin composition according to claim 4, wherein the epoxy resin is represented by the following formula (3) and has an epoxy equivalent weight of 500 to 10000 g/eq: 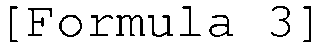 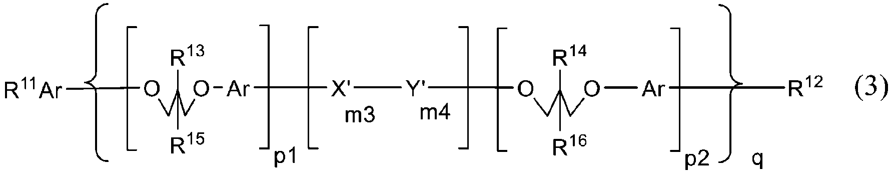
wherein each Ar is independently a structure having an aromatic ring that is unsubstituted or has a substituent;
X' is a structural unit represented by the following general formula (3-1), and Y' is a structural unit represented by the following general formula (3-2), 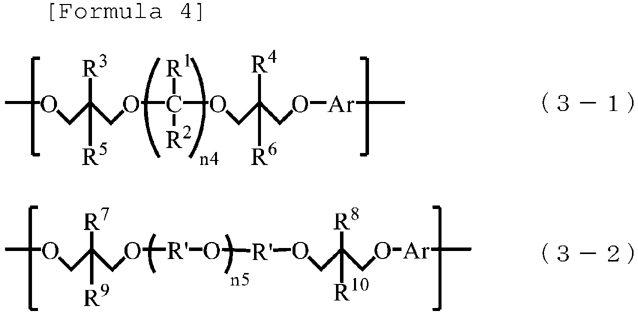
wherein Ar is as defined above,
R¹ and R² are each independently a hydrogen atom, a methyl group, or an ethyl group,
R' is a divalent hydrocarbon group having 2 to 12 carbon atoms,
R³, R⁴, R⁷, and R⁸ are each independently a hydroxyl group, a glycidyl ether group, or a 2-methylglycidyl ether group,
R⁵, R⁶, R⁹, and R¹⁰ are each independently a hydrogen atom or a methyl group,
n4 is an integer of 2 to 16, and
n5 is an average number of repeating units and is 2 to 30;
R1¹ and R¹² are each independently a glycidyl ether group or a 2-methylglycidyl ether group;
R¹³ and R¹⁴ are each independently a hydroxyl group, a glycidyl ether group, or a 2-methylglycidyl ether group;
R¹⁵ and R¹⁶ are each a hydrogen atom or a methyl group; m3, m4, p1, p2, and q are each an average number of repetitions,
m3 and m4 are each independently 0 to 25 and m3 + m4 ≥ 1,
p1 and p2 are each independently 0 to 5, and
q is 0.5 to 5,
provided that bonding between X' represented by the general formula (3-1) and Y' represented by the general formula (3-2) is optionally random or block, and total numbers of the structural units X' and Y' in one molecule are m3 and m4, respectively.

7. The curable resin composition according to claim 6, wherein the epoxy resin is represented by the following formula (4): 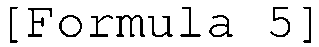 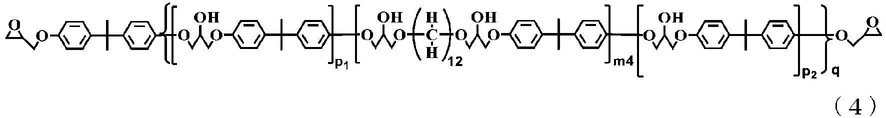 wherein p1, p2, q, and m4 are each an average number of repetitions, and independently, p1 is 0 to 5, p2 is 0 to 5, q is 0.5 to 5, and m4 is 0 to 25.

8. A curable resin composition, wherein the curable resin composition according to claim 2 is a self-repairable composition or a composition for remolding materials.

9. A cured article made by curing the curable resin composition according to claim 2.

10. A laminated product comprising:
a substrate; and
a layer including the cured article according to claim 9.

11. A heat-resistant member comprising the cured article according to claim 9.

12. A method for producing an amino group-containing compound, the method comprising synthesizing in situ an amino group-containing compound represented by the formula (1) in a process of curing with a compound (I) having reactivity with the amino group-containing compound, using a dienophile intermediate represented by the following general formula (1)':

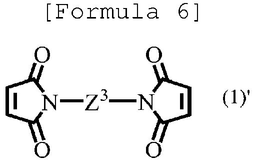

13. A cured article obtained by a curing reaction using the formula (1)', an anthracene having an amino group, and a compound (I) having reactivity with the amino group-containing compound as essential components.
